# EUROPÄISCHE PATENTANMELDUNG

(11) **EP 2 103 589 A1**
(43) Veröffentlichungstag der Anmeldung: **23.09.2009**
(21) Anmeldenummer: 08010867.3
(22) Anmeldetag: 20.02.2003
(51) Int. Cl.: C07C 25/18, C09K 19/12

(54) **Polyphenylene als Flüssigkristalle**

(30) Priorität: 15.03.2002 DE 10211597
(62) Teilanmeldung aus: 03003811.1
(71) Anmelder: Merck Patent GmbH, 64293 Darmstadt (DE)
(72) Erfinder: Pauluth, Detlef, Dr., 64372 Ober-Ramstadt (DE); Kirsch, Peer, Dr., Tsuzuki-ku Yokohama (JP); Bäuerle, Peter, Prof. Dr., 89275 Thalfingen (DE); Deeg, Oliver, Dr., 67157 Wachenheim (DE)

(57) **Zusammenfassung**

Die vorliegende Erfindung betrifft ein Verfahren zur Herstellung von Ringverbindungen über eine kombinatorische Synthese, die auf einer Suzuki-Kupplung, einer anschließenden Halo-Demetallierung und abschließend einer Suzuki-Kupplung beruht. Die Suzuki-Kupplungen werden jeweils mit einer Boronsäure oder einem Boronsäureester durchgeführt. Ebenso betrifft die vorliegende Erfindung die entsprechenden Ringverbindungen sowie die hierfür verwendeten neuen Synthesebausteine. Die erfindungsgemäßen Ringverbindungen finden vorzugsweise Verwendung als Bestandteile in flüssigkristallinen Mischungen.

## Beschreibung

Die vorliegende Erfindung betrifft ein Verfahren zur Herstellung von Ringverbindungen über eine kombinatorische Synthese, die auf einer Suzuki-Kupplung, einer anschließenden Halo-Demetallierung und abschließend einer Suzuki-Kupplung beruht. Die Suzuki-Kupplungen werden jeweils mit einer Boronsäure oder einem Boronsäureester durchgeführt. Ebenso betrifft die vorliegende Erfindung die entsprechenden Ringverbindungen sowie die hierfür verwendeten neuen Synthesebausteine. Die erfindungsgemäßen Ringverbindungen finden vorzugsweise Verwendung als Bestandteile in flüssigkristallinen Mischungen.

Aus dem Stand der Technik ist die Palladium-katalysierte Kreuzkupplungsreaktion von aromatischen Borverbindungen, wie Boronsäuren und deren Derivaten sowie aromatischen Halogenverbindungen bekannt (EP 0 470 795 A1) und findet seit einigen Jahren auch verstärkt Anwendung im Bereich der organischen Synthese. Das in der EP 0 470 795 A1 beschriebene Verfahren, beruht auf einem homogenkatalysierten Prozess unter Verwendung von Palladium(0)-Komplexen, insbesondere Tetrakis-(Triphenylphosphan) Palladium(0). Nachteilig an diesem Verfahren ist, dass die Komplexe oxidationsempfindlich sind, wodurch sie an Aktivität verlieren. Aufgrund der variierenden Aktivität ist das Verfahren schlecht reproduzierbar und sind die Ausbeuten zum Teil sehr niedrig. Darüber hinaus sind die Komplexe sehr teuer.

Aus der DE 44 26 671 A1 ist ein Verfahren zur Herstellung mehrkerniger aromatischer Verbindungen durch Kreuzkupplung von aromatischen Borverbindungen mit aromatischen Halogenverbindungen oder Perfluoralkylsulfonaten unter Palladium-Katalyse in Gegenwart mindestens eines wasserlöslichen Komplexliganden bekannt. Hierbei wird mit einem 2-Phasen-System aus einer organischen und einer wässrigen Phase gearbeitet, wobei der Palladiumkatalysator in der organischen Phase gelöst vorliegt. Nachteilig an diesem Verfahren ist, dass eine sehr gute Durchmischung der beiden Phasen Voraussetzung für die Umsetzung ist. Darüber hinaus ist auch dieses Verfahren aufgrund der eingesetzten Katalysatoren sehr teuer.

Ausgehend von diesem Stand der Technik kann es als Aufgabe der vorliegenden Erfindung angesehen werden, ein Verfahren bereitzustellen, bzw. eine kombinatorische Synthesestrategie zu entwerfen, mit der bei geringer Anzahl an Reaktionsschritten Produkte mit hoher Ausbeute und hohem Umsatz erzielt werden können.

Erfindungsgemäß wird diese Aufgabe durch das Verfahren mit den Merkmalen des Anspruchs 1 sowie durch die flüssigkristallinen Ringverbindungen mit den Merkmalen des Anspruchs 14 gelöst, wobei als Synthesebausteine Boronsäuren bzw. Boronsäureester mit den Merkmalen der Ansprüche 17 und 19 eingesetzt werden. Die abhängigen Ansprüche zeigen bevorzugte Ausführungsformen auf.

Erfindungsgemäß wird ein Verfahren zur Herstellung von Ringverbindungen der allgemeinen Formeln I bis IV bereitgestellt.

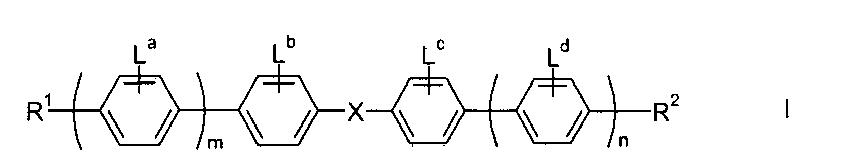

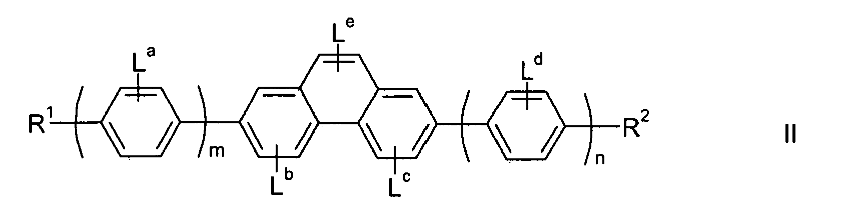

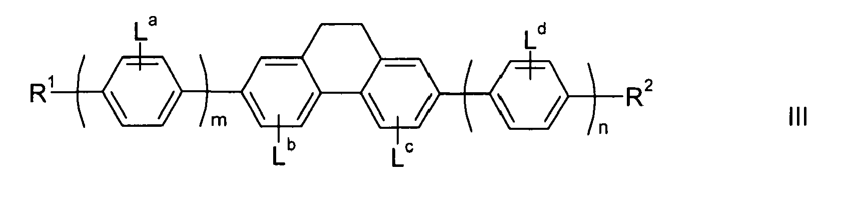

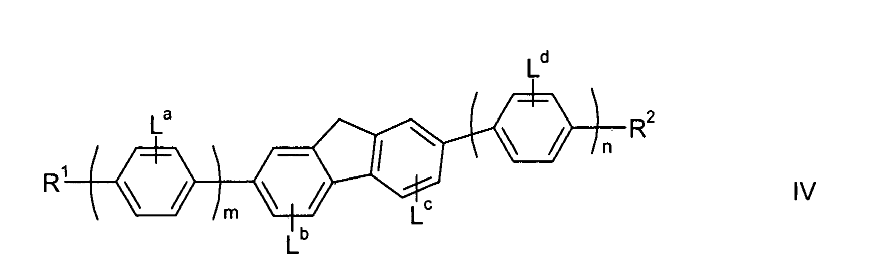

Dabei können m und n unabhängig voneinander, gleich oder verschieden, die Werte 0 oder 1 annehmen, wobei die Summe (m + n) 1 oder 2 ist. Besonders bevorzugt ist m = n = 1, d.h. die Summe (m + n) = 2.
X ist eine Einfachbindung, -CH₂-CH₂-, -CH=CH-, -C≡C- oder 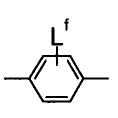 Vorzugsweise ist X eine Einfachbindung.

L ist unabhängig voneinander, gleich oder verschieden R, F, Cl, Br, I, OH, OR, SH, SR, CN, NO₂, NO, CHO, COOH, COOR, CONH₂, CONHR, CONR₂, CF₃, NH₂, NHR oder NR₂, wobei R eine Alkyl-, eine Alkenyl- bzw. eine Acylgruppe mit 1 bis 12 C-Atomen oder eine Arylgruppe mit 6 C-Atomen, die gegebenenfalls wiederum durch eine Alkylgruppe mit 1 bis 12 C-Atomen substituiert sein kann, darstellt. Bevorzugt ist L unabhängig voneinander, gleich oder verschieden F, Cl, CF₃ und CH₃, wobei F besonders bevorzugt ist.

Die Indizes a, b, c, d, e und f können unabhängig voneinander, gleich oder verschieden die Werte 0, 1 oder 2 annehmen, wobei die Summe a+b+c+d+e+f Werte zwischen 1 und 8, vorzugsweise zwischen 3 und 8 und besonders bevorzugt zwischen 4 und 8 annimmt. In den aromatischen Ringsystemen der Formeln I bis IV kann dabei eine oder zwei CH-Gruppen durch N ersetzt sein.

R¹ und R² sind unabhängig voneinander, gleich oder verschieden H, F, Cl, CN oder NCS. Ebenso ist es möglich, dass R¹ und/oder R² einen geradkettigen oder verzweigten, gegebenenfalls chiralen Alkylrest oder Alkoxyrest mit 1 bis 12 C-Atomen, bzw. einen Alkenylrest oder Alkinylrest mit 2 bis 8 C-Atomen darstellen, wobei in diesen organischen Resten auch jeweils eine CH₂-Gruppe durch -O-, -CO-, -O-CO- oder -COO- so ersetzt sein können, dass Heteroatome nicht direkt miteinander verknüpft sind und/oder ein oder mehrere H durch Halogen, vorzugsweise F, ersetzt sein können. Bevorzugt stellen R¹ und/oder R² einen geradkettigen Alkylrest mit 1 bis 7 C-Atomen dar. In einer weiteren bevorzugten Ausführungsform ist R² ein chiraler Alkylrest mit 1 bis 12 C-Atomen.

Für den Fall, dass m = 0 oder n = 0 ist, ist vorzugsweise eine CH₂-Gruppe in R¹ oder R² durch eine der folgenden Gruppen ersetzt:
a) trans-1,4-Cyclohexylen, worin auch eine oder mehrere nicht benachbarte CH₂-Gruppen durch -O- und/oder -S- ersetzt sein können,
b) einen Rest aus der Gruppe 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl oder
c) 1,4-Cyclohexenylen.

Die Reste a), b), c) können dabei auch durch CN und/oder Halogen substituiert sein.

Falls R¹ und/oder R² in den vor- und nachstehenden Formeln einen Alkylrest bedeutet, so kann dieser geradkettig oder verzweigt sein. Besonders bevorzugt ist er geradkettig, hat 1, 2, 3, 4, 5, 6 oder 7 C-Atome und bedeutet demnach Methyl, Ethyl, Propyl, Butyl, Pentyl, Hexyl oder Heptyl, ferner Octyl, Nonyl, Decyl, Undecyl oder Dodecyl.

Falls R¹ und/oder R² einen Alkylrest bedeutet, in dem eine CH₂-Gruppe durch -O- ersetzt ist, so kann dieser geradkettig oder verzweigt sein. Vorzugsweise ist er geradkettig und hat 1 bis 10 C-Atome. Besonders bevorzugt ist die erste CH₂-Gruppe dieses Alkylrestes durch -O- ersetzt, so dass der Rest R¹ die Bedeutung Alkoxy erhält und Methoxy, Ethoxy, Propoxy, Butoxy, Pentyloxy, Hexyloxy, Heptyloxy, Octyloxy, Nonyloxy bedeutet.

Weiterhin kann auch eine CH₂-Gruppe an anderer Stelle durch -O- ersetzt sein, so dass der Rest R¹ und/oder R² vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl bedeutet.

Falls R¹ und/oder R² einen Alkylrest bedeutet, in dem eine CH₂-Gruppe durch -O- und eine durch -CO- ersetzt ist, so sind diese benachbart. Somit beeinhalten diese eine Acyloxygruppe -CO-O- oder eine Oxycarbonylgruppe -O-CO-. Besonders bevorzugt sind diese geradkettig und haben 2 bis 6 C-Atome.

Sie bedeuten demnach insbesondere Acetyloxy, Propionyloxy, Butyryl-oxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, But-oxycarbonylmethyl, 2-(Methoxycarbonyl)ethyl, 2-(Ethoxycarbonyl)ethyl, 2-(Propoxycarbonyl)-ethyl, 3-(Methoxycarbonyl)propyl, 3-(Ethoxycarbonyl)propyl und 4-(Methoxycarbonyl)butyl.

Falls R¹ und/oder R² einen einfach durch CN oder CF₃ substituierten Alkyl- oder Alkenylrest bedeutet, so ist dieser Rest vorzugsweise geradkettig und die Substitution durch CN oder CF₃ in ω-Position.

Falls R¹ und/oder R² einen mindestens einfach durch Halogen substituierten Alkylrest bedeutet, so ist dieser Rest vorzugsweise geradkettig. Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I mit verzweigter Flügelgruppe R¹ und/oder R² können gelegentlich wegen einer besseren Löslichkeit in den üblichen, flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verzweigte Gruppen dieser Art enthalten in der Regel nicht mehr als eine Kettenverzweigung. Bevorzugte verzweigte Reste R¹ und/oder R² sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methyl-pentyl, 2-Ethylhexyl, 2-Propylpentyl, Isopropoxy, 2-Methylpropoxy, 2-Methylbutoxy, 3-Methylbutoxy, 2-Methylpentyloxy, 3-Methylpentyloxy, 2-Ethylhexyloxy, 1-Methylhexyloxy und 1-Methylheptyloxy.

Nachstehend haben m, n, X, L, a, b, c, d, e, f, R¹ und R² die oben angegebene Bedeutung, sofern nicht ausdrücklich etwas anderes vermerkt ist. Entsprechend können in den nachstehend genannten Ringsystemen analog den Ringverbindungen der allgemeinen Formeln I bis IV auch eine oder zwei CH-Gruppen durch N ersetzt sein.

Die Herstellung der Ringverbindungen der allgemeinen Formeln I bis IV geht aus von den Verbindungen der Formeln V bis VIII:

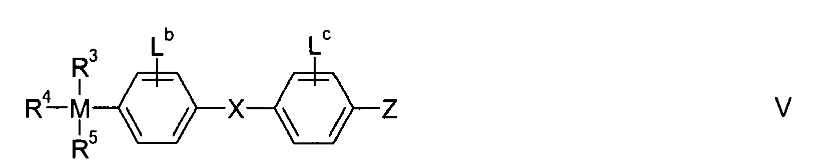

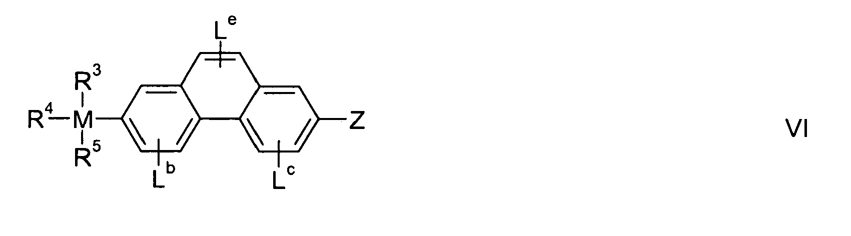

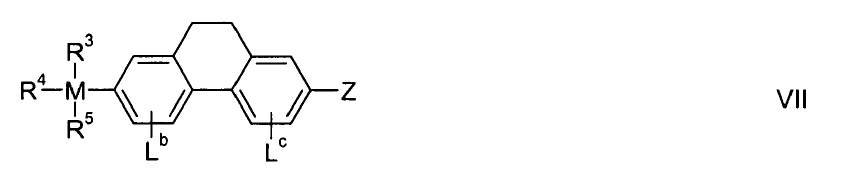

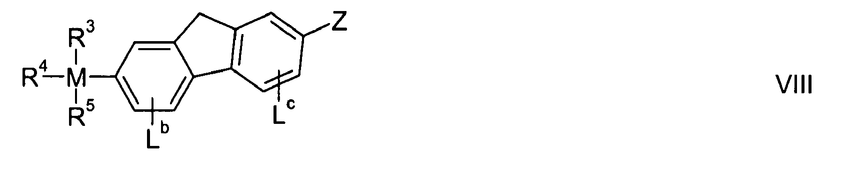

Z ist dabei ausgewählt aus der Gruppe I, Cl, Br und OTf (Triflat), wobei Z bevorzugt I ist, und M ist ausgewählt aus der Gruppe Si, Ge und Sn, wobei Si bevorzugt ist.

R³, R⁴ und R⁵ sind unabhängig voneinander, gleich oder verschieden H, C₁-C₁₂-Alkyl oder C₁-C₁₂-Alkoxy.

Ferner kann mindestens einer der Reste R³, R⁴ und R⁵ in den allgemeinen Formeln V bis VIII ein fluorhaltiger Alkylrest der allgemeinen Formel IX sein,

-(CH₂)ₚ-(CF₂)-_{q}-CF₃ IX

wobei p Werte im Bereich von 2 bis 4, q Werte ≥ 2 und die Summe (p + q) Werte im Bereich von 2 bis 11 annehmen kann. Bevorzugt ist q > p.

Nachstehend haben R³, R⁴, R⁵, M und Z die oben angegebene Bedeutung, sofern nicht ausdrücklich etwas anderes vermerkt ist.

Die Herstellung der Ringverbindungen erfolgt über eine kombinatorische Synthese, bei der in einer matrixartigen Anordnung von Reaktionsgefäßen die folgenden Reaktionsschritte durchgeführt werden:
A) Suzuki-Kupplung mit einer Boronsäure oder einem Boronsäureester, vorzugsweise einem Boronsäureester, der allgemeinen Formel X 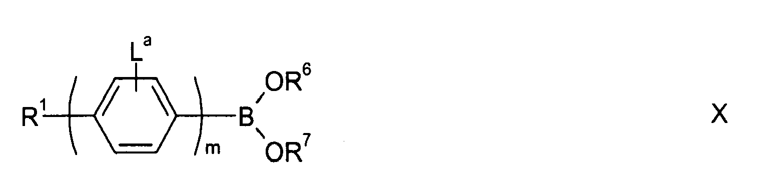 wobei R⁶ und R⁷ unabhängig voneinander, gleich oder verschieden H, C₁-C₁₂-Alkyl, C₂-C₁₂-Alkenyl, vorzugsweise C₃-C₁₂-Alkenyl, oder C₆-Aryl ist. R⁶ und R⁷ können auch cyclisch verbrückt sein.
   Nachstehend haben R⁶ und R⁷ die oben angegebene Bedeutung, sofern nicht ausdrücklich etwas anderes vermerkt ist.
B) anschließende Halo-Demetallierung, vorzugsweise Jodo-Desilylierung, und
C) eine Suzuki-Kupplung mit einer Boronsäure oder einem Boronsäureester, vorzugsweise einem Boronsäureester, der allgemeinen Formel XI.

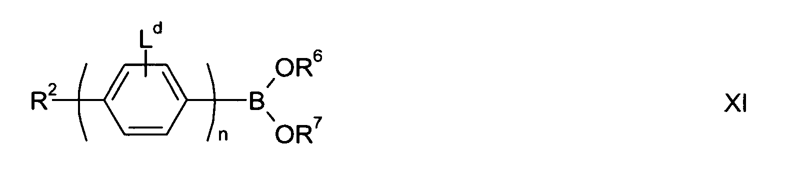

In einem Schritt A) vorgelagerten Schritt wird die Verbindung der Formel V, bei der X eine Einfachbindung darstellt, bevorzugt aus einer Boronsäure oder einem Boronsäureester der Formel XII 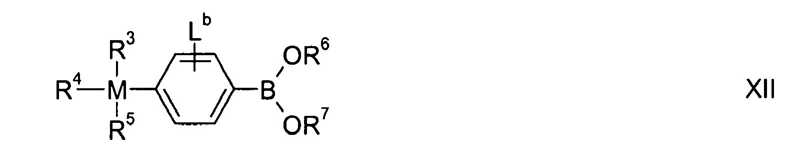 durch eine als kombinatorische Synthese in einer matrixartigen Anordnung von Reaktionsgefäßen durchgeführten Suzuki-Kupplung mit einem zumindest teilweise fluorierten p-Bromjodbenzol und einem sich anschließenden Jodierungsschritt zur Substitution des Broms, z.B. mittels Butyllithium und einem Jodierungsmittel, hergestellt.

Ein besonderer Vorteil des erfindungsgemäßen Verfahrens ist es, dass dieses als kombinatorische Synthese ausgeführt wird. Für die Entwicklung der kombinatorischen Synthese ist es erforderlich, ein Synthesekonzept mit aufeinander abgestimmten Reaktionen auszuarbeiten, wobei die Reaktionen hinsichtlich Umsatz und Ausbeute zu optimieren sind. Das Verfahren wird als divergente Synthese durchgeführt, was gegenüber einer linearen Synthese den Vorteil hat, dass lediglich drei Reaktionsschritte benötigt werden. Die lineare Synthese würde dagegen fünf Reaktionsschritte benötigen. Ein weiterer Vorteil liegt in dem geringeren Reinigungsaufwand gegenüber der Durchführung eines linearen Syntheseverfahrens.

Vorzugsweise wird eine Aufreinigung der Zwischenprodukte und/oder Endprodukte durch Umkristallisation und die Isolierung der Kristalle über Kartuschen für die Festphasenextraktion durchgeführt, wobei die Aufreinigung parallel für alle Reaktionsgefäße erfolgt.

Hinsichtlich der Suzuki-Kupplungen wird die hierfür benötigte Base vorzugsweise ausgewählt aus der Gruppe der Hydroxide, Carbonate und Fluoride, wobei Bariumhydroxid und Caesiumfluorid besonders bevorzugt sind.

Als Katalysator wird vorzugsweise eine palladiumhaltige Verbindung, besonders bevorzugt Palladiumacetat eingesetzt.

Die Reaktion wird vorzugsweise in einem polaren Lösungsmittel, wie z.B. einem Alkohol oder Ether, durchgeführt. Besonders bevorzugt wird dabei Isopropanol verwendet.

In einer weiteren besonders bevorzugten Variante der Suzuki-Kupplung wird als Base Caesiumfluorid, als Katalysator Palladiumacetat und als Lösungsmittel Dioxan eingesetzt.

Die hier beschriebenen bevorzugten Varianten der Suzuki-Kupplung haben den Vorteil, dass ein nahezu quantitativer Umsatz erreicht wird und das Produkt palladiumfrei ist. Gleichzeitig treten keine nicht-abtrennbaren Nebenprodukte auf, so dass die Reinheit der Rohprodukte für die Folgereaktionen ausreichend ist. Weitere Vorteile dieses Katalysators sind dabei seine einfache Handhabung, seine Luftstabilität sowie sein niedriger Preis.

Die Suzuki-Kupplungen werden vorzugsweise bei einer Temperatur zwischen 10 und 120°C und einer Reaktionsdauer zwischen 0,1 und 30 Stunden durchgeführt. Besonders bevorzugt sind Temperaturen zwischen 50 und 100°C und eine Reaktionsdauer zwischen 18 und 24 Stunden.

Die Jodo-Desilylierung, als bevorzugte Variante der Halo-Demetallierung, wird vorzugsweise unter Zusatz von Jodchlorid in Methylcyanid durchgeführt. Dabei werden vorzugsweise Temperaturen zwischen 10 und 75°C und eine Reaktionsdauer zwischen 0,1 und 20 Stunden eingehalten. Besonders bevorzugt sind Temperaturen zwischen 20 und 30°C und eine Reaktionsdauer zwischen 0,5 und 2 Stunden.

Ein wichtiger Schritt in der Synthese ist die Unterdrückung von Seitenkettenchlorierungen während der Halo-Demetallierung. Diese Seitenkettenchlorierung der Reste R¹ und R² kann bei der Durchführung einer Jodo-Desilylierung in Acetonitril nahezu vollständig vermieden werden. Ein weiterer Grund für die bevorzugte Stellung der Jodo-Desilylierung in Verbindung mit der Suzuki-Kupplung ist deren Kompatibilität gegenüber o-Fluor-Substituenten.

Das erfindungsgemäße Verfahren zur Herstellung von Ringverbindungen der allgemeinen Formeln I bis IV wird im folgenden schematisch ausführlich für die bevorzugten Ringverbindungen der allgemeinen Formel XVIII dargestellt.

Die Herstellung des Biphenyls, das Ausgangssubstanz des erfindungsgemäßen Verfahrens ist, wird in Schema 1 dargestellt. Schema 2 zeigt die Synthese des Terphenyls ausgehend vom Biphenyl aus Schema 1 und einer Boronsäure (Schritt A des erfindungsgemäßen Verfahrens). In Schema 3 wird die Jodo-Desilylierung des Terphenyls aus Schema 2 (Schritt B des erfindungsgemäßen Verfahrens) und in Schema 4 die Synthese des Quarterphenyls (Schritt C des erfindungsgemäßen Verfahrens) beschrieben.

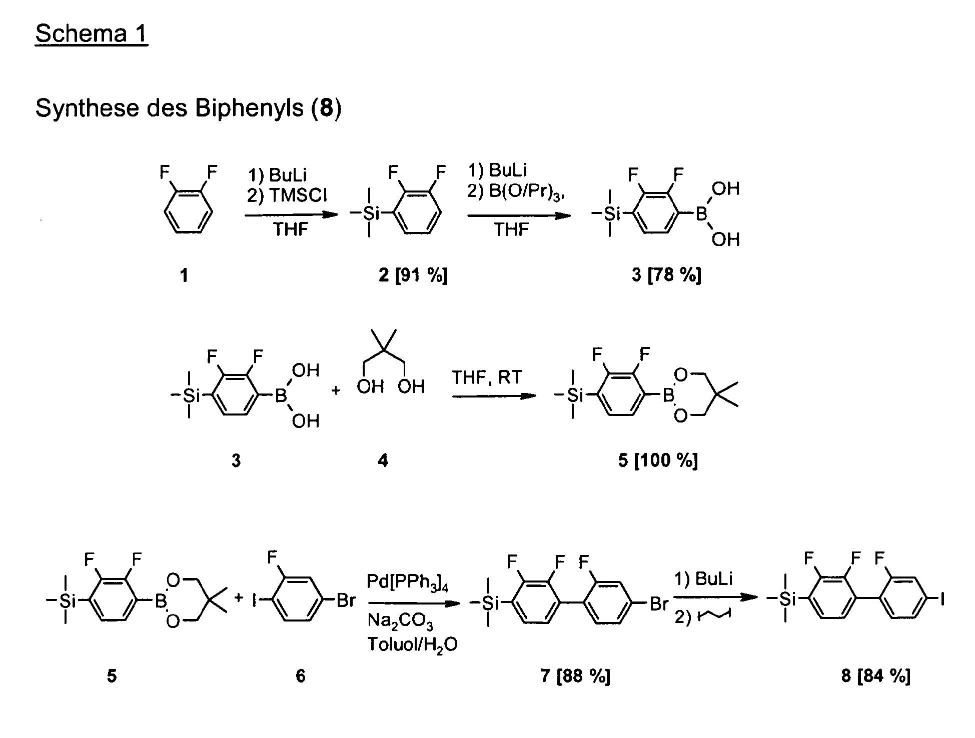

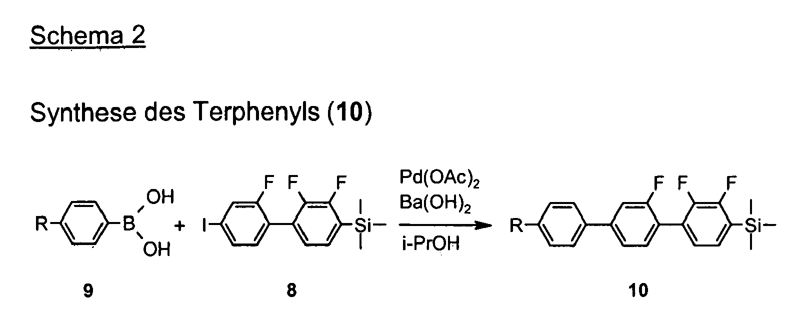

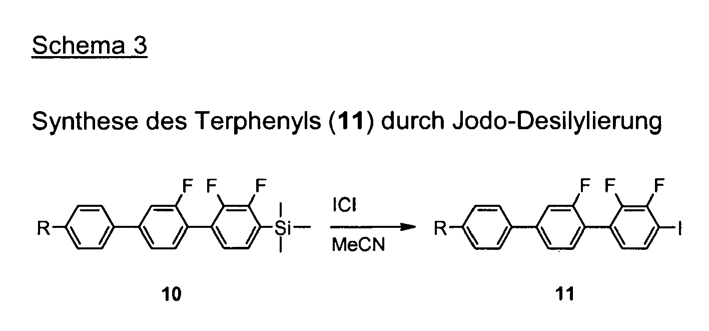

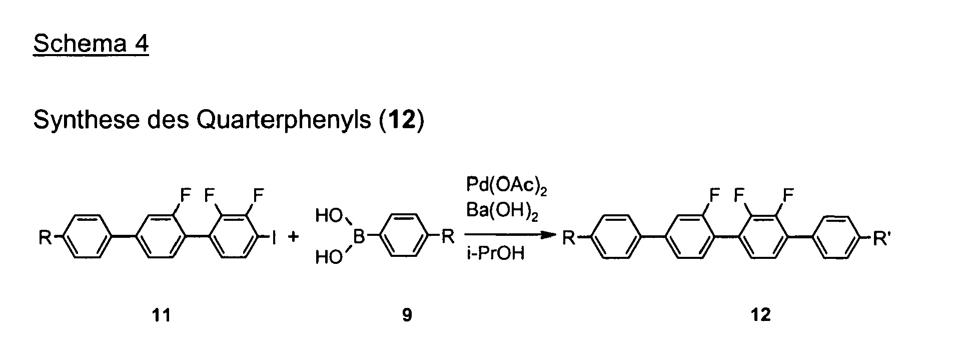

Erfindungsgemäß werden ebenso Ringverbindungen der allgemeinen Formel XIII bereitgestellt, 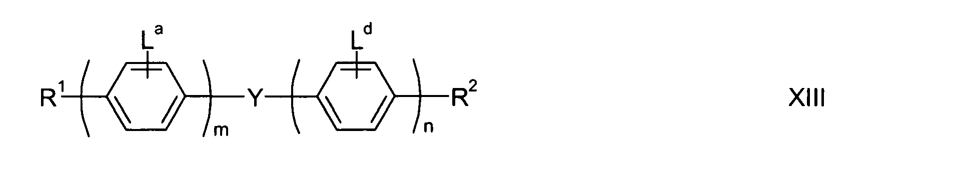 wobei m und n unabhängig voneinander, gleich oder verschieden, die Werte 0 oder 1 annehmen, wobei die Summe (m + n) 1 oder 2 ist. Bevorzugt ist m = n = 1, d.h. die Summe (m + n) = 2.

Y stellt dabei eine Gruppe der allgemeinen Formeln XIV bis XVII dar. 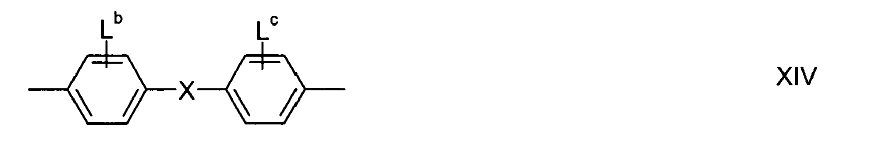 mit X = Einfachbindung, -CH=CH-, -C≡C- oder

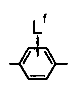

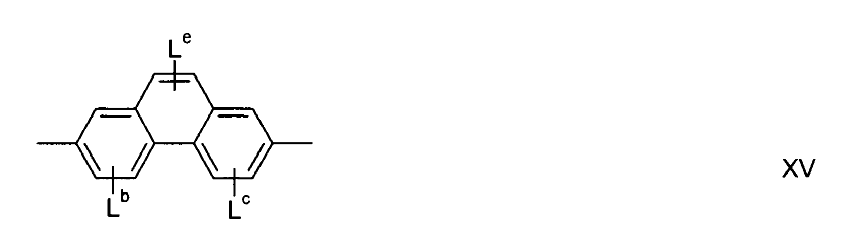

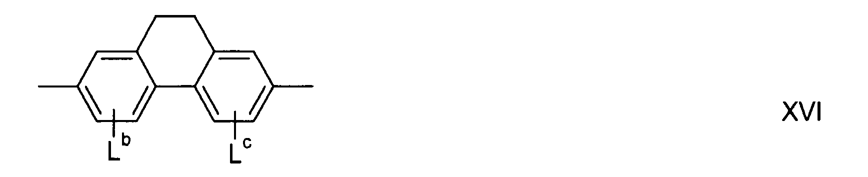

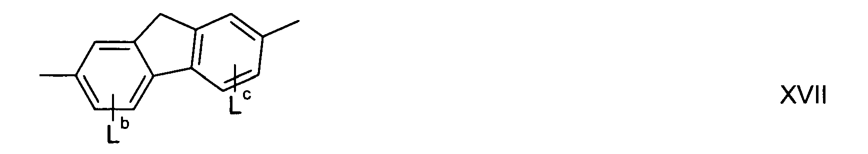

L ist unabhängig voneinander, gleich oder verschieden R, F, Cl, Br, I, OH, OR, SH, SR, CN, NO₂, NO, CHO, COOH, COOR, CONH₂, CONHR, CONR², CF₃, NH₂, NHR oder NR², wobei R eine Alkyl-, eine Alkenyl- bzw. eine Acylgruppe mit 1 bis 12 C-Atomen oder eine Arylgruppe mit 6 C-Atomen, die gegebenenfalls wiederum durch eine Alkylgruppe mit 1 bis 12 C-Atomen substituiert sein kann, darstellt. Bevorzugt ist L unabhängig voneinander, gleich oder verschieden F, Cl, CF₃ und CH₃, wobei F besonders bevorzugt ist.

Die Indizes a, b, c, d, e und f können unabhängig voneinander, gleich oder verschieden die Werte 0, 1 oder 2 annehmen, wobei die Summe (a+b+c+d+e+f) Werte zwischen 1 und 8, vorzugsweise zwischen 3 und 8 und besonders bevorzugt zwischen 4 und 8 annimmt. In den aromatischen Ringsystemen der Formeln XIII bis XVII kann dabei eine oder zwei CH-Gruppen durch N ersetzt sein.

R¹ und R² sind unabhängig voneinander, gleich oder verschieden H, F, Cl, CN oder NCS. Ebenso ist es möglich, dass R¹ und/oder R² einen geradkettigen oder verzweigten, gegebenenfalls chiralen Alkylrest oder Alkoxyrest mit 1 bis 12 C-Atomen, bzw. einen Alkenylrest oder Alkinylrest mit 2 bis 8 C-Atomen darstellen, wobei in diesen organischen Resten auch jeweils eine CH₂-Gruppe durch -O-, -CO-, -O-CO-, -COO- oder -CH=CH-so ersetzt sein können, dass Heteroatome nicht direkt miteinander verknüpft sind und/oder ein oder mehrere H durch Halogen, vorzugsweise F, ersetzt sein können.

Für den Fall, dass m = 0 oder n = 0 ist, muß eine CH₂-Gruppe in R¹ oder R² durch eine der folgenden Gruppen ersetzt sein:
a) trans-1,4-Cyclohexylen, worin auch eine oder mehrere nicht benachbarte CH₂-Gruppen durch -O- und/oder -S- ersetzt sein können,
b) einen Rest aus der Gruppe 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl oder
c) 1,4-Cyclohexenylen.

Die Reste a), b), c) können dabei auch durch CN und/oder Halogen substituiert sein.

Bevorzugt sind Ringverbindungen der allgemeinen Formel XVIII, 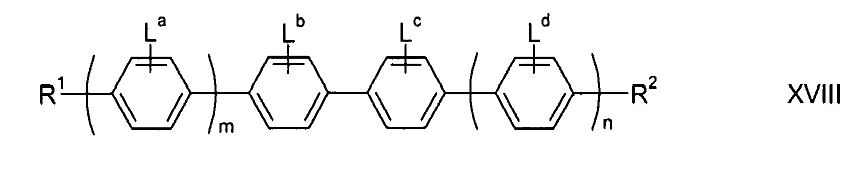 wobei dabei besonders bevorzugt m = n = 1 ist und die Reste R¹ und R² unabhängig voneinander, gleich oder verschieden, ausgewählt sind aus der Gruppe C₁-C₇-Alkyl.

Besonders bevorzugt sind dabei Ringverbindungen der folgenden Formeln XVIIIa bis XVIIIg: 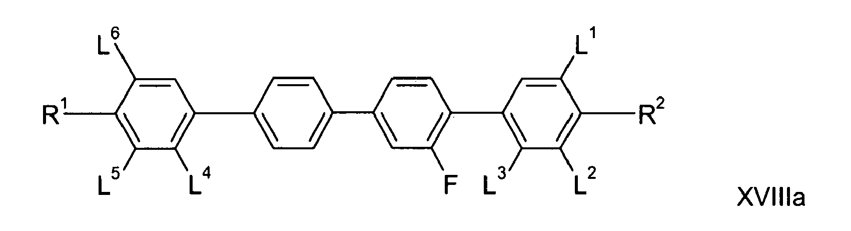 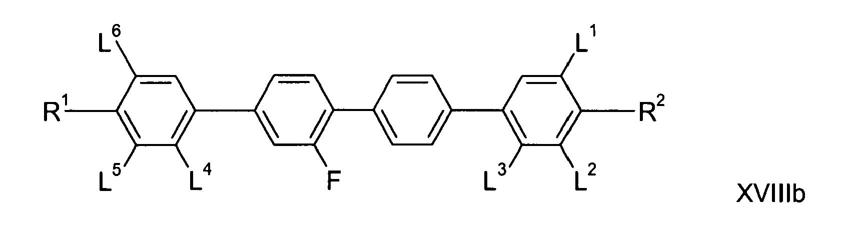 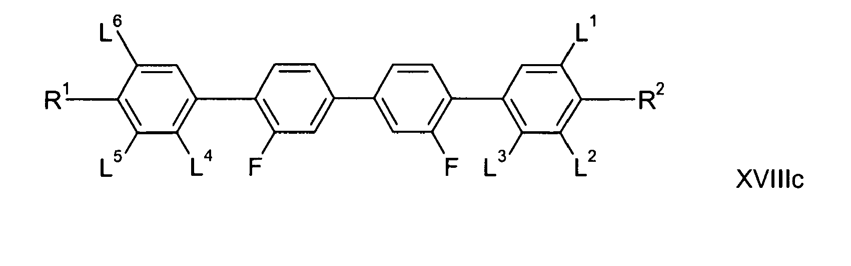 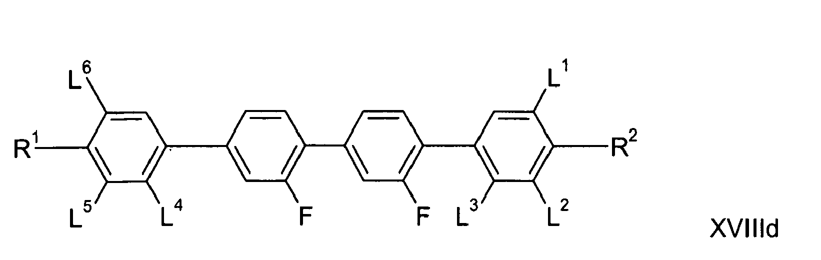 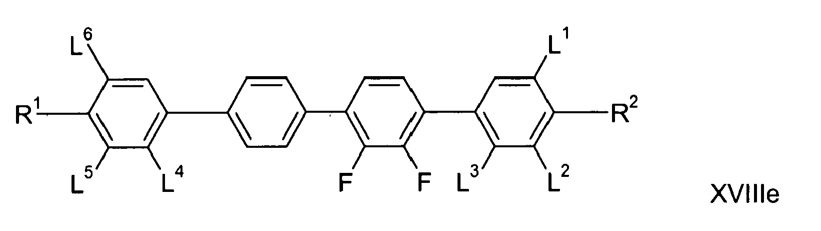 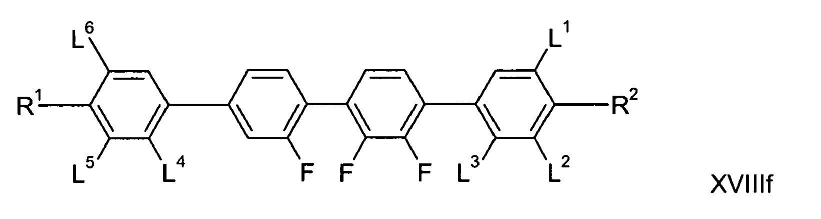 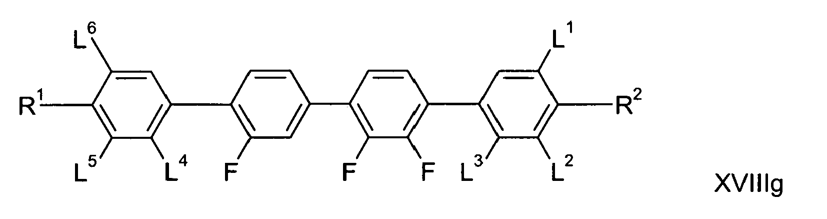 wobei R¹ und R² die oben in Bezug auf Formel XVIII angegebenen Bedeutungen, L¹, L² und L³ die Bedeutungen von L^{d} und L⁴, L⁵ und L⁶ die Bedeutungen von L^{a} annehmen können. Insbesondere bevorzugt sind dabei die Ringverbindungen der Formeln XVIIIf und XVIIIg.

Erfindungsmäß werden auch Boronsäuren oder Boronsäureester der allgemeinen Formel XIX als Synthesebausteine bereitgestellt.

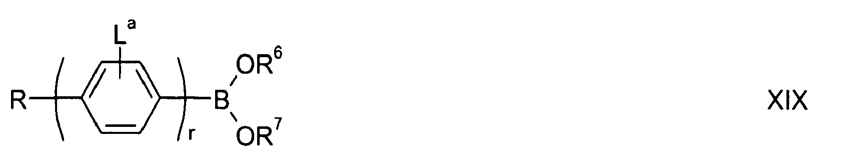

Vorzugsweise weisen die Boronsäuren oder Boronsäureester eine Struktur gemäß der allgemeinen Formel XX auf

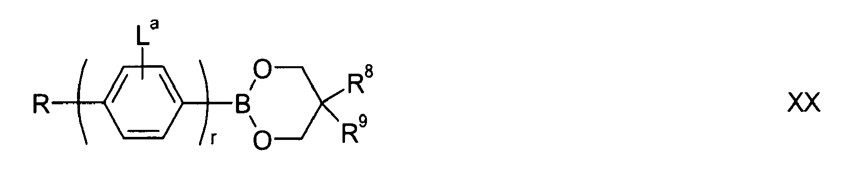

In den Strukturen XIX und XX kann r = m oder n sein und damit die Werte 1 oder 2, vorzugsweise 1, annehmen. Wenn r = m ist, dann ist R = R¹ und wenn r = n ist, dann ist R = R².

R kann die Bedeutungen von R¹ und R² haben und ist damit H, F, Cl, CN oder NCS.

Ebenso ist es möglich, dass R einen geradkettigen oder verzweigten, gegebenenfalls chiralen Alkylrest oder Alkoxyrest mit 1 bis 12 C-Atomen, bzw. einen Alkenylrest oder Alkinylrest mit 2 bis 8 C-Atomen darstellt, wobei in diesem organischen Rest auch eine CH₂-Gruppe durch -O- oder -COO- so ersetzt sein kann, dass Heteroatome nicht direkt miteinander verknüpft sind und/oder ein oder mehrere H durch F ersetzt sein können. Darüber hinaus kann für den Fall, dass r = 0 ist, eine CH₂-Gruppe in R auch durch eine der folgenden Gruppen ersetzt sein:
a) trans-1,4-Cyclohexylen, worin auch eine oder mehrere nicht benachbarte CH₂-Gruppen durch -O- und/oder -S- ersetzt sein können,
b) einen Rest aus der Gruppe 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl oder
c) 1,4-Cyclohexenylen.

R⁶, R⁷, L und a haben dabei die oben angegebenen Bedeutungen.

R⁸ und R⁹ bedeuten unabhängig voneinander, gleich oder verschieden C₁-C₁₂-Alkyl oder C₆-Aryl.

Erfindungsgemäß werden ferner Boronsäuren oder Boronsäureester der allgemeinen Formel XXI bereitgestellt.

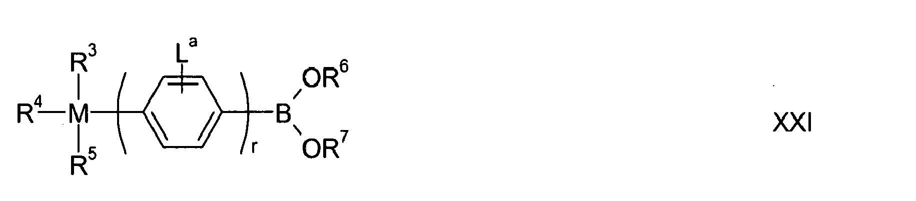

Bevorzugt weisen die Boronsäuren oder Boronsäureester die allgemeine Formel XXII auf,

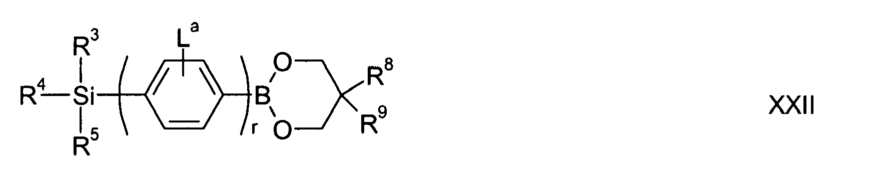

M, r, R³, R⁴, R⁵, R⁶, R⁷, R⁸, R⁹, L und a haben dabei die oben angegebenen Bedeutungen.

Ebenso können in den aromatischen Ringsystemen der allgemeinen Formeln XIX, XX, XXI und XXII eine oder zwei CH-Gruppen durch N ersetzt sein.

Die Erfindung wird im folgenden anhand von Ausführungsbeispielen näher beschrieben, in denen beispielhafte, erfindungsgemäße Verbindungen aufgeführt werden, die durch kombinatorische Synthese hergestellt worden sind, ohne dadurch jedoch in irgend einer Weise eingeschränkt zu werden.

Vor- und nachstehend bedeuten Prozentangaben Gewichtsprozent.

Gleichzeitig wurden diese Verbindungen anhand ihrer Phasenübergänge charakterisiert.

K bedeutet kristalliner Zustand, N = nematische Phase, Sm = smektische Phase und I = isotrope Phase. Die Angaben zwischen diesen Symbolen stellen die Übergangstemperaturen dar. Alle Temperaturen sind in Grad Celsius angegeben.

### Beispiel 1

### Herstellung der Boronsäure (4)

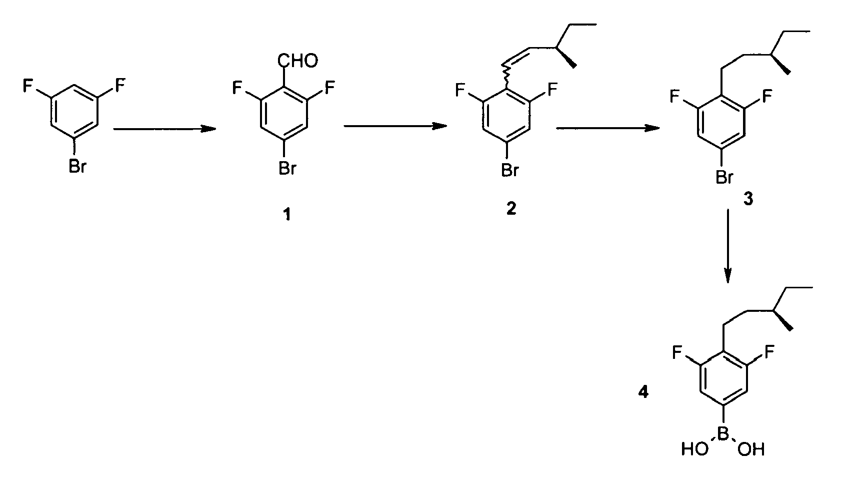

### 4-Brom-2,6-difluor-benzaldehyd (1)

Zu einer Lösung von 19,3 g (0,1 mmol) 1-Brom-3,5-difluorbenzol in 120 ml getrocknetem Tetrahydrofuran wird bei -70°C unter Rühren 55 ml (0,11 mmol) 2 M Lithiumdiisopropylamid zugegeben. Nach 30 Minuten wird *N-*Formylpiperidin bei dieser Temperatur zugetropft. Man läßt auf 0°C erwärmen. Bei ca. 0°C wird das Reaktionsgemisch in kaltes Wasser gegossen, mit 10 % HCl angesäuert und zweimal mit Methyl-tert-butylether extrahiert. Die vereinigten organischen Phasen werden mit Wasser gewaschen, über Na₂SO₄ getrocknet, filtriert und das Lösungsmittel im Vakuum entfernt. Der Rückstand wird über SiO₂ filtriert (Heptan/Dichlormethan 1:1). (Ausbeute: 17,6 g, 78 %) 5-Brom-1,3-difluor-2-[(R)-3-methyl-pent-1-enyl]-benzol (2)

### Phosphoniumsalz Synthese

10 g (66 mmol) *S*-(+)-1-brom-2-methylbutan und 17,4 g (66 mmol) Triphenylphosphin werden in 50 ml Toluol gelöst und bei 110°C 48 Stunden gerührt. Man läßt auf Raumtemperatur erwärmen, dann wird der Feststoff filtriert und mit Toluol nachgewaschen (6,9 g, 25 %).

Das Phosphoniumsalz (6,9 g, 16,7 mmol) wird in 25 ml getrocknetem Tetrahydrofuran suspendiert und auf 0 bis 5°C abgekühlt. Bei dieser Temperatur werden 8,3 ml (16,7 mmol) 2 M Lithiumdiisopropylamid zugetropft. Nach 15 Minuten wird eine Lösung von 3,8 g (16,7 mmol) **1** in 25 ml getrocknetem Tetrahydrofuran zugetropft. Man läßt auf Raumtemperatur erwärmen und 1 Stunde bei dieser Temperatur rühren. Anschließend wird Wasser zugegeben, mit 10 % HCl angesäuert und zweimal mit Methyl-tert-butylether extrahiert. Die vereinigten organischen Phasen werden mit Wasser gewaschen, über Na₂SO₄ getrocknet, filtriert und das Lösungsmittel im Vakuum entfernt. Der Rückstand wird über SiO₂ filtriert (Heptan), (Ausbeute: 1,8 g, 22 %).

### 5-Brom-1,3-difluor-2-[(R)-3-methyl-pentyl]-benzol (3)

Eine Lösung von 1,8 g (5,9 mmol) **2** in 50 ml Heptan wird mit 0,4 g Pt-C-5 % (trocken) versetzt und bei Normaldruck 20 Stunden hydriert. Das Lösungsmittel wird im Vakuum entfernt und die gesamte Menge zu **4** umgesetzt.

### 3,5-Difluor-4-[((R)-3-methyl-pentyl)-phenyl]-phenylboronsäure (4)

Zu einer Lösung von 1,4 g (5 mmol) **3** in 5 ml trockenem Diethylether werden bei -78°C 3,3 ml (5,5 mmol) 1,6 M BuLi zugetropft. Nach 30 Minuten werden 0,6 ml (5,5 mmol) Trimethylborat zugetropft. Man läßt über Nacht auf Raumtemperatur erwärmen. Anschließend werden 5,2 ml Wasser, 5,2 ml Methyl-tert-butylether und 3 ml konz. HCl zugegeben. Die organische Phase wird mit Wasser (2 x 3 ml) und ges. NaCl (1 x 3 ml) gewaschen, mit Magnesiumsulfat getrocknet und das Lösungsmittel im Vakuum entfernt. Der Rückstand wird über SiO₂ filtriert (Heptan/Dichlormethan 1:1), (Ausbeute: 0,8 g, 70 %).

### Beispiel 2

### Herstellung des Biphenyls (8)

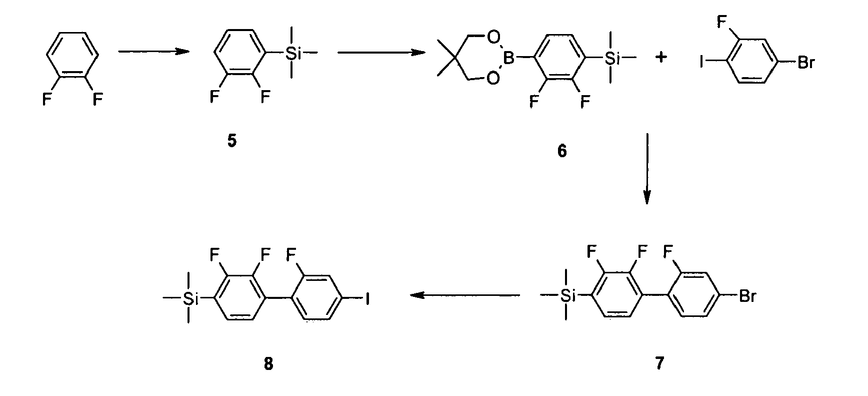

### 1,2-Difluor-3-trimethylsilyl-benzol (5)

Zu einer Lösung von 114 g (1 mol) 1,2-Difluor-benzol in 1 I trockenem Tetrahydrofuran werden bei -78°C 625 ml (1 mol) 1,6 M BuLi zugetropft. Nach 1 Stunde werden bei -78°C 140 ml (120 g, 1,1 mol) Trimethylsilylchlorid langsam zugetropft. Man läßt über Nacht auf Raumtemperatur erwärmen und gibt dann je 200 ml Methyl-tert-butylether und Wasser zu. Die organische Phase wird mit Wasser (2 x 100 ml) und ges. NaCl (1 x 100 ml) gewaschen, mit Magnesiumsulfat getrocknet und das Lösungsmittel im Vakuum entfernt. Aus dem Rückstand destillieren bei 100 bis 102°C/70 mbar 170 g (91 %) 5 über.

### 2-(2,3-Difluor-4-trimethylsilyl-phenyl)-5,5-dimethyl-[1,3,2]dioxaborinan (6)

Zu einer Lösung von 169 g (910 mmol) 1,2-Difluor-3-trimethylsilyl-benzol (5) in 1,4 I trockenem Tetrahydrofuran wird bei -78°C 625 ml (1 mol) 1,6 M BuLi zugetropft. Nach 15 Minuten werden 276 ml (1,2 mol) Triisopropylborat zugetropft. Man läßt über Nacht auf Raumtemperatur erwärmen. Anschließend werden 200 ml Wasser, 200 ml Methyl-tert-butylether und 100 ml konz. HCl zugegeben. Die organische Phase wird mit Wasser (2 x 100 ml) und ges. NaCl (1 x 100 ml) gewaschen, mit Magnesiumsulfat getrocknet und das Lösungsmittel im Vakuum entfernt. Der ölige Rückstand wird in 270 ml THF gelöst und 94 g (910 mmol) Neopentylglykol sowie 455 g Magnesiumsulfat zugegeben. Nach 1 Stunde Rühren wird das Lösungsmittel bei reduziertem Druck entfernt und der ölige Rückstand mit Petrolether versetzt (273 ml). Ausgefallenes Neopentylglykol wird durch Filtration entfernt. Aus dem Filtrat kristallisiert bei -25°C 251 g (87 %) **6.**

### (4'-Brom-2,3,2'-trifluor-biphenyl-4-yl)-trimethyl-silan (7)

Zu einer Lösung von 15 g (50 mmol) 4-Brom-2-fluor-1-jod-benzol, 14,9 g (50 mmol) **6** und 2,31 g (2 mmol) [Pd(PPh₃)₄] in 100 ml Dioxan wird eine Lösung von 16,6 g (120 mmol) K₂CO₃ in 50 ml Wasser gegeben und über Nacht unter Rückfluß gerührt. Die organische Phase wird mit Wasser und ges. NaCl gewaschen, über MgSO₄ getrocknet und über SiO₂ filtriert. Das Lösungsmittel wird bei reduziertem Druck entfernt und der ölige Rückstand mit Petrolether versetzt und bei -25°C umkristallisiert (Ausbeute: 88 %, Smp. 78,0°C).

### Trimethyl-(2,3,2'-trifluor-4'-jod-biphenyl-4-yl)-silan (8)

Zu einer Lösung von 15,8 g (44 mmol) 7 in 132 ml trockenem THF wird bei -78°C 30 ml (48 mmol) 1,6 M BuLi zugetropft. Nach 15 Minuten werden 16 g (57,2 mmol) 1,2-Dijodethan als Feststoff zugegeben und man läßt innerhalb von 1 Stunde auf Raumtemperatur erwärmen. Anschließend werden Wasser und Methyl-tert-butylether zugegeben. Die organische Phase wird mit Wasser, ges. Na₂S₂O₅ und ges. NaCl gewaschen, über MgSO₄ getrocknet und über SiO₂ filtriert. Das Lösungsmittel wird bei reduziertem Druck entfernt und der ölige Rückstand mit Petrolether versetzt und bei -25°C umkristallisiert (Ausbeute: 84 %, Smp. 76,0°C).

### Beispiel 3

### Herstellung des Terphenyls (10)

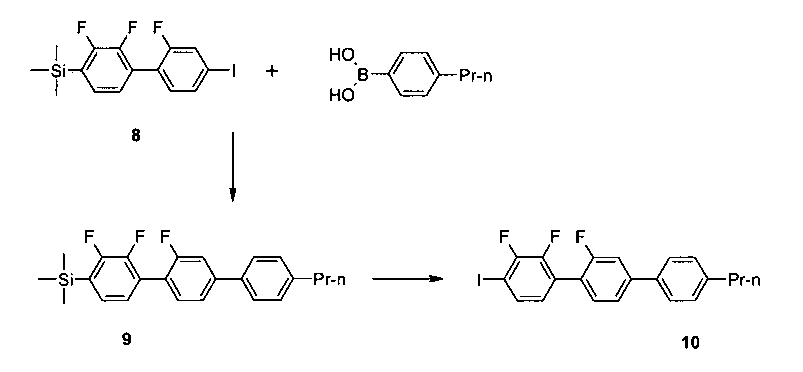

### Trimethyl-(2,3,2'-trifluor-4"-propyl-[1,1';4',1"]terphenyl-4-yl)-silan (9)

Eine Lösung von (2 mmol) **8 ,** (2,2 mmol) 4-Propyl-phenylboronsäure, 250 mg (2,40 mmol) Neopentylglykol, 1,53 g (4,84 mmol) Ba(OH)₂ · 8 H₂O, 1,35 ml (0,103 mmol, 5 mol %) einer 76 mM acetonischen Pd(OAc)₂-Lösung in 20 ml 95% i-PrOH wird 12 Stunden bei 80°C gerührt. Anschließend wird das Lösungsmittel bei reduziertem Druck entfernt. Zu dem Rückstand werden 5 ml 2 M HCl gegeben und mit Dichlormethan (3 x 5 ml) extrahiert. Die vereinigten organischen Phasen werden über MgSO₄ getrocknet und über SiO₂ filtriert. Das Lösungsmittel wird bei reduziertem Druck entfernt und der Rückstand zu **10** umgesetzt (Ausbeute: 89 %).

### 2,3,2'-Trifluor-4-jodo-4"-propyl-[1, 1';4', 1 "]terphenyl (10)

Die gesamte Menge des Terphenyls **9** wird in 4 ml absolutem Acetonitril gelöst und mit 1,2 ml (6 mmol) einer 5 M Lösung von ICI in Acetonitril zur Reaktion gebracht und 1 Stunde bei Raumtemperatur gerührt. Der Rückstand wird bei -20°C in 2 Stunden kristallisiert. Anschließend wird die überstehende Lösung abgesaugt und das zurückbleibende Produkt mit 2 M Na₂S₂O₅ (10 ml) und Wasser (10 ml) gewaschen und im Ölpumpenvakuum getrocknet (Ausbeute: 54 %).

### Beispiel 4

### Herstellung des Quarterphenyls (11)

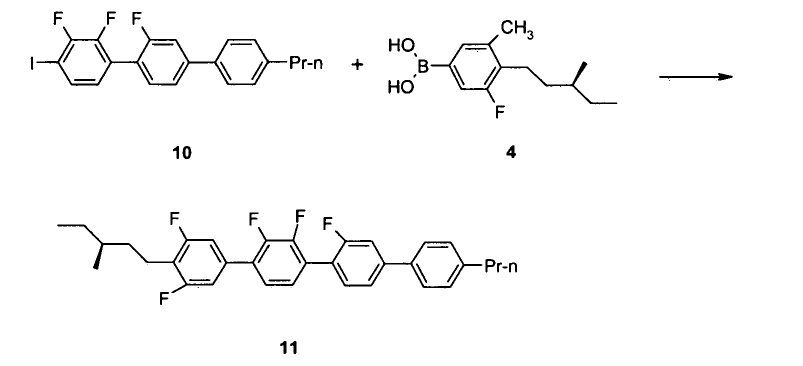

### 3,5,2',3',2"-Pentafluor-4"'-methyl-4-((S)-3-methyl-pentyl)-[1,4';1',1";4",1''']quarterphenyl (11)

Eine Lösung von (0,1 mmol) **10**, (0,12 mmol) **4**, 14 mg (0,13 mmol) Neopentylglykol, 95 mg (0,3 mmol) Ba(OH)₂ · 8 H₂O, 80 µl (6,08 mmol, 5 mol %) einer 76 mM acetonischen Pd(OAc)₂-Lösung in 2 ml 95 % i-PrOH wird 12 Stunden bei 80°C gerührt. Anschließend wird das Lösungsmittel bei reduziertem Druck entfernt. Zu dem Rückstand werden 2 ml 2 M HCl gegeben und mit Dichlormethan (3 x 2 ml) extrahiert. Die vereinigten organischen Phasen werden über MgSO₄ getrocknet und über SiO₂ filtriert. Das Lösungsmittel wird bei reduziertem Druck entfernt und der Rückstand durch zweimalige Umkristallisation aus Nonan gereinigt (Ausbeute: 60 %).

Zu einer kommerziell erhältlichen, nematischen Basismischung MLC-6260 der Fa. Merck KGaA, Darmstadt, wird 1 Gew.-% des Quarterphenyls (**11**) hinzugegeben und die Drehkraft HTP der Zusammensetzung nach der Grandjean-Cano Methode bei 20°C bestimmt. Die Zusammensetzung weist eine HTP von -1,9 auf.

### Beispiel 5

### Herstellung des Quarterphenyls (13)

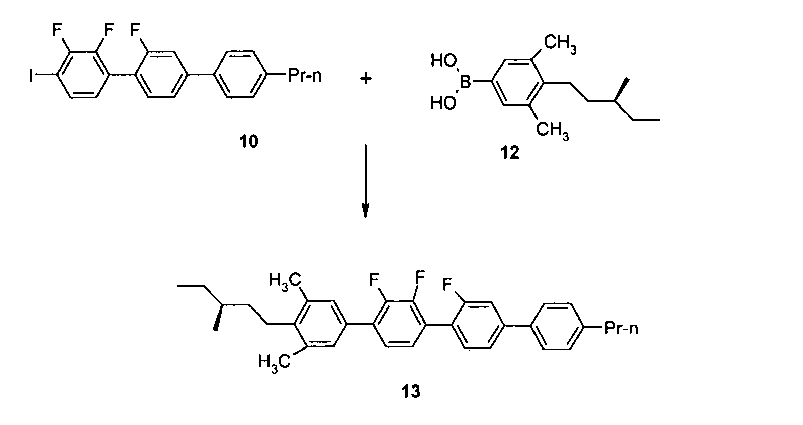

### 2',3',2"-Trifluor-3,5-dimethyl-4-((S)-3-methyl-pentyl)-4"'-propyl-[1,4';1',1";4",1"']quarterphenyl (13)

Eine Lösung von (0,1 mmol) **10,** (0,12 mmol) **12**, 14 mg (0,13 mmol) Neopentylglykol, 95 mg (0,3 mmol) Ba(OH)₂ · 8 H₂O, 80 µl (6,08 mmol, 5 mol %) einer 76 mM acetonischen Pd(OAc)₂-Lösung in 2 ml 95% i-PrOH wird 12 Stunden bei 80°C gerührt. Anschließend wird das Lösungsmittel bei reduziertem Druck entfernt. Zu dem Rückstand werden 2 ml 2 M HCl gegeben und mit Dichlormethan (3 x 2 ml) extrahiert. Die vereinigten organischen Phasen werden über MgSO₄ getrocknet und über SiO₂ filtriert. Das Lösungsmittel wird bei reduziertem Druck entfernt und der Rückstand durch zweimalige Umkristallisation aus Nonan gereinigt (Ausbeute: 65 %).

Zu einer kommerziell erhältlichen, nematischen Basismischung MLC-6260 der Fa. Merck KGaA, Darmstadt, wird 1 Gew.-% des Quarterphenyls (13) hinzugegeben und die Drehkraft HTP der Zusammensetzung nach der Grandjean-Cano Methode bei 20°C bestimmt. Die Zusammensetzung weist eine HTP von -1,5 auf.

Zu einer kommerziell erhältlichen, nematischen Basismischung MJ-001667 der Fa. Merck KGaA, Darmstadt, wird 1 Gew.-% des Quarterphenyls (**13**) hinzugegeben und die Drehkraft HTP der Zusammensetzung nach der Grandjean-Cano Methode bei 20°C bestimmt. Die Zusammensetzung weist eine HTP von -1,9 auf.

### Beispiel 6

### Herstellung des Quarterphenyls (15)

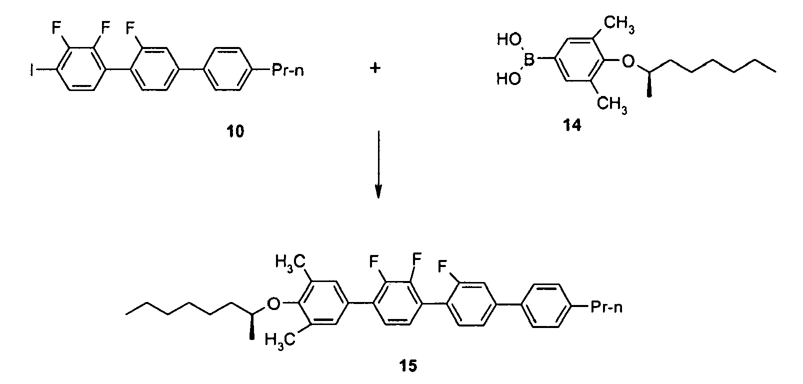

### 2',3',2"-Trifluor-3,5-dimethyl-4-((S)-1-methyl-heptyloxy)-4"'-propyl-[1,4';1',1";4",1''']quarterphenyl (15)

Eine Lösung von (0,1 mmol) **10,** (0,12 mmol) **14**, 14 mg (0,13 mmol) Neopentylglykol, 95 mg (0,3 mmol) Ba(OH)₂ · 8 H₂O, 80 µl (6.08 mmol, 5 mol %) einer 76 mM acetonischen Pd(OAc)₂-Lösung in 2 ml 95 % i-PrOH wird 12 Stunden bei 80°C gerührt. Anschließend wird das Lösungsmittel bei reduziertem Druck entfernt. Zu dem Rückstand werden 2 ml 2 M HCl gegeben und mit Dichlormethan (3 x 2 ml) extrahiert. Die vereinigten organischen Phasen werden über MgSO₄ getrocknet und über SiO₂ filtriert. Das Lösungsmittel wird bei reduziertem Druck entfernt und der Rückstand durch zweimalige Umkristallisation aus Nonan gereinigt (Ausbeute: 68 %).

Zu einer kommerziell erhältlichen, nematischen Basismischung MLC-6260 der Fa. Merck KGaA, Darmstadt, wird 1 Gew.-% des Quarterphenyls (**15**) hinzugegeben und die Drehkraft HTP der Zusammensetzung nach der Grandjean-Cano Methode bei 20°C bestimmt. Die Zusammensetzung weist eine HTP von -12,1 auf.

Zu einer kommerziell erhältlichen, nematischen Basismischung MJ-001667 der Fa. Merck KGaA, Darmstadt, wird 1 Gew.-% des Quarterphenyls (**15**) hinzugegeben und die Drehkraft HTP der Zusammensetzung nach der Grandjean-Cano Methode bei 20°C bestimmt. Die Zusammensetzung weist eine HTP von -19,5 auf.

Analog der Beispiele 1 bis 6 sowie der Reaktionsschemata 1 bis 4 werden unter Einsatz der entsprechenden Ausgangsverbindungen die Verbindungen der folgenden Beispiele hergestellt:

### Beispiele 7 bis 21

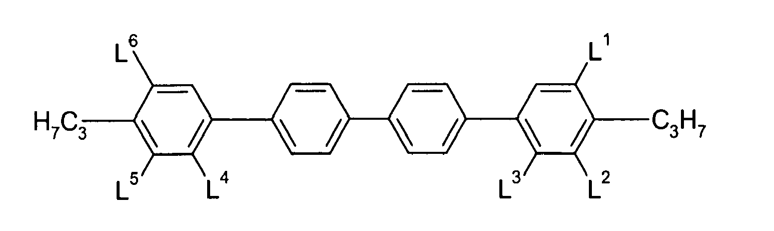

**Tabelle 1**

| Beispiel | L¹ | L² | L³ | L⁴ | L⁵ | L⁶ | Phasenübergänge [°C] |
|---|---|---|---|---|---|---|---|
| 7 | H | H | H | H | H | H | |
| 8 | H | F | H | H | H | H | K 264 SmE 276 SmA 351 N 355 I |
| 9 | H | H | F | H | H | H | K 190 Sm? 210 SmC 237 SmA 250 N 335,2 I |
| 10 | F | F | H | H | H | H | K 158 SmE 243 SmA 326 I |
| 11 | H | F | F | H | H | H | K 197 SmC 218 SmA 300 N 332,5 I |
| 12 | H | F | H | F | H | H | K 191 SmC 202 SmA 263 N 304,7 I |
| 13 | H | F | H | H | F | H | K 239 SmA 322 I |
| 14 | H | H | F | F | H | H | K 174 N 297,5 I |
| 15 | F | F | H | F | H | H | K 145 SmA 270 N 274 I |
| 16 | F | F | H | H | F | H | K 190 SmA 292 I |
| 17 | H | F | F | F | H | H | K 183 N 290,8 I |
| 18 | H | F | F | H | F | H | K 177 SmA 282 N 300,1 I |
| 19 | F | F | H | F | F | H | K 154 SmA 265 N 269 I |
| 20 | F | F | H | H | F | F | K 203 SmA 249 N 250,6 I |
| 21 | H | F | F | F | F | H | K 178 SmC 206 SmA 219 N 284,4 I |

### Beispiele 22 bis 46

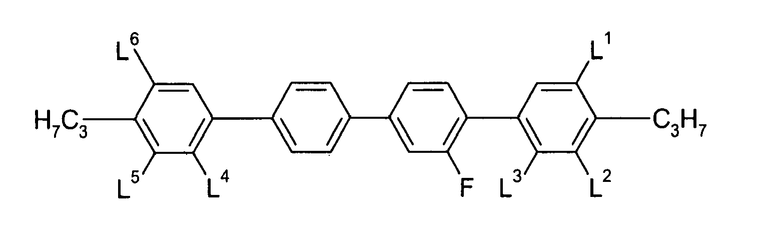

**Tabelle 2**

| Beispiel | L¹ | L² | L³ | L⁴ | L⁵ | L⁶ | Phasenübergänge [°C] |
|---|---|---|---|---|---|---|---|
| 22 | H | H | H | H | H | H | K 146 Sm? 184 SmC 198 SmA 283 N 336,8 I |
| 23 | H | F | H | H | H | H | K 161 SmC 165 SmA 293 N 313,7 I |
| 24 | H | H | F | H | H | H | K 128 SmC 162 SmA 186 N 297,6 I |
| 25 | H | H | H | F | H | H | K 127 N 297,8 I |
| 26 | H | H | H | H | F | H | K 152 SmA 227 N 307,0 I |
| 27 | F | F | H | H | H | H | K 105 Sm? 152 SmA 284 N 287,2 I |
| 28 | H | F | F | H | H | H | K 130 SmC (119) SmA 232 N 295,5 I |
| 29 | H | H | H | F | F | H | |
| 30 | H | H | H | H | F | F | K 152 SmA 268 N 274 I |
| 31 | H | F | H | H | F | H | K 139 SmA 266 N 283,9 I |
| 32 | H | F | H | F | H | H | K 106 SmC 106 SmA 198 N 272,1 I |
| 33 | H | H | F | H | F | H | K 97 SmC 144 SmA 153 N 264,5 I |
| 34 | H | H | F | F | H | H | K 120 N 257,7 I |
| 35 | F | F | H | F | H | H | K 95 SmA 226 N 245,0 I |
| 36 | F | F | H | H | F | H | K 118 SmA 243 N 255,7 I |
| 37 | H | F | F | F | H | H | K 129 N 251,0 I |
| 38 | H | F | F | H | F | H | K 112 SmC (110) SmA 211 N 260,1 I |
| 39 | H | F | H | H | F | F | K 129 SmA 242 N 251,0 I |
| 40 | H | H | F | H | F | F | K 122 SmA 197 N 231,5 I |
| 41 | H | F | H | F | F | H | |
| 42 | H | H | F | F | F | H | |
| 43 | F | F | H | H | F | F | K 158 SmA 206 N 223,7 I |
| 44 | F | F | H | F | F | H | |
| 45 | H | F | F | H | F | F | K 124 SmA 208 N 228,4 I |
| 46 | H | F | F | F | F | H | |

### Beispiele 47 bis 71

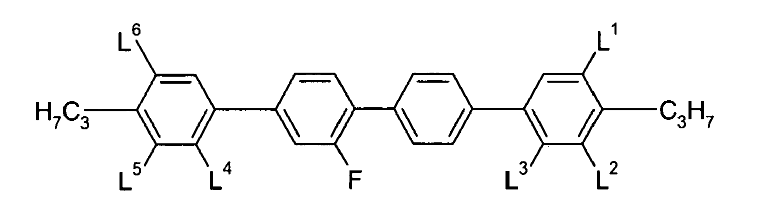

**Tabelle 3**

| Beispiel | L¹ | L² | L³ | L⁴ | L⁵ | L⁶ | Phasenübergänge [°C] |
|---|---|---|---|---|---|---|---|
| 47 | H | H | H | H | H | H | K 186 Sm? 188 N 330,8 I |
| 48 | H | F | H | H | H | H | K 144 SmC (141) SmA 251 N 297,9 I |
| 49 | H | H | F | H | H | H | K 118 N 295,1 I |
| 50 | H | H | H | F | H | H | K 112 N 299,7 I |
| 51 | H | H | H | H | F | H | K 148 SmA 246 N 299,4 I |
| 52 | F | F | H | H | H | H | K 150 SmA 260 N 267,9 I |
| 53 | H | F | F | H | H | H | K 133 SmA 152 N 287,6 I |
| 54 | H | H | H | F | F | H | K 124 SmA 147 N 289,1 I |
| 55 | H | H | H | H | F | F | K 136 SmA 260 N 264,0 I |
| 56 | H | F | H | H | F | H | K 125 SmA 249 N 271,8 I |
| 57 | H | F | H | F | H | H | K 121 N 264,5 I |
| 58 | H | H | F | H | F | H | K 128 N 262,6 I |
| 59 | H | H | F | F | H | H | K 131 N 267,1 I |
| 60 | F | F | H | F | H | H | K 123 SmA 182 N 225,9 I |
| 61 | F | F | H | H | F | H | K 152 SmA 240 N 242,0 I |
| 62 | H | F | F | F | H | H | K 146 N 256,1 I |
| 63 | H | F | F | H | F | H | K 136 SmA 187 N 256,6 I |
| 64 | H | F | H | H | F | F | K 151 SmA 243 N 243,0 I |
| 65 | H | H | F | H | F | F | K 141 SmA 181 N 226,3 I |
| 66 | H | F | H | F | F | H | K 129 SmC 174 SmA 186 N 257,7 I |
| 67 | H | H | F | F | F | H | K 146 N 254,8 I |
| 68 | F | F | H | H | F | F | K 191 SmA 219 I |
| 69 | F | F | H | F | F | H | K 133 SmA 206 N 225,1 I |
| 70 | H | F | F | H | F | F | K 145 SmA 208 N 224,5 I |
| 71 | H | F | F | F | F | H | K 159 N 245,4 I |

### Beispiele 72 bis 86

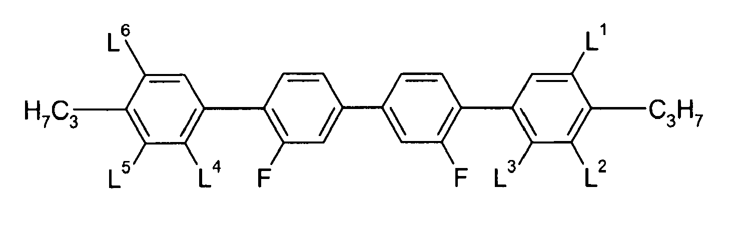

**Tabelle 4**

| Beispiel | L¹ | L² | L³ | L⁴ | L⁵ | L⁶ | Phasenübergänge |
|---|---|---|---|---|---|---|---|
| 72 | H | H | H | H | H | H | K 131 SmC (113) SmA 186 N 293,6 I |
| 73 | H | F | H | H | H | H | K 96 SmA 223 N 270,3 I |
| 74 | H | H | F | H | H | H | K 129 N 256,5 I |
| 75 | F | F | H | H | H | H | K 117 SmA 226 N 244,4 I |
| 76 | H | F | F | H | H | H | K 126 N 254,4 I |
| 77 | H | F | H | F | H | H | K 134 N 234,6 I |
| 78 | H | F | H | H | F | H | K 120 SmA 217 N 250,0 I |
| 79 | H | H | F | F | H | H | K 156 N 222,6 I |
| 80 | F | F | H | F | H | H | K 145 SmA 167 N 209,7 I |
| 81 | F | F | H | H | F | H | K 150 SmA 202 N 224,7 I |
| 82 | H | F | F | F | H | H | K 155 N 214,8 I |
| 83 | H | F | F | H | F | H | K 141 SmA 160 N 242,0 I |
| 84 | F | F | H | F | F | H | K 159 SmA 172 N 203,8 I |
| 85 | F | F | H | H | F | F | K 190 N 205,0 I |
| 86 | H | F | F | F | F | H | K 162 N 214,8 I |

### Beispiele 87 bis 111

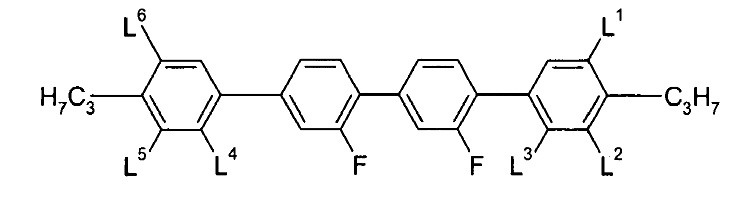

**Tabelle 5**

| Beispiel | L¹ | L² | L³ | L⁴ | L⁵ | L⁶ | Phasenübergänge [°C] |
|---|---|---|---|---|---|---|---|
| 87 | H | H | H | H | H | H | K 118 N 296,01 I |
| 88 | H | F | H | H | H | H | K 98 SmA 197 N 269,1 I |
| 89 | H | H | F | H | H | H | K 128 N 262,1 I |
| 90 | H | H | H | F | H | H | K 134 N 265,1 I |
| 91 | H | H | H | H | F | H | K 111 SmA 167 N 263,8 I |
| 92 | F | F | H | H | H | H | K 109 SmA 222 N 238,6 I |
| 93 | H | F | F | H | H | H | K 143 N 249,2 I |
| 94 | H | H | H | F | F | H | K 139 N 253,0 I |
| 95 | H | H | H | H | F | F | K 129 SmA 205 N 229,7 I |
| 96 | H | F | H | H | F | H | K 120 SmA 205 N 244,1 I |
| 97 | H | F | H | F | H | H | K 139 N 238,5 I |
| 98 | H | H | F | H | F | H | K 136 N 232,1 I |
| 99 | H | H | F | F | H | H | K 157 N 234,3 I |
| 100 | F | F | H | F | H | H | K 149 SmA 160 N 207,8 I |
| 101 | F | F | H | H | F | H | K 128 SmA 204 N 220,1 I |
| 102 | H | F | F | F | H | H | K 162 N 222,2 I |
| 103 | H | F | F | H | F | H | K 151 N 224,5 I |
| 104 | H | F | H | H | F | F | K 134 SmA 204 N 217,1 I |
| 105 | H | H | F | H | F | F | K 161 N 200,7 I |
| 106 | H | F | H | F | F | H | K 152 SmA 148 N 229,0 I |
| 107 | H | H | F | F | F | H | K 164 N 220,4 I |
| 108 | F | F | H | H | F | F | K 170 SmA 190 N 199,2 I |
| 109 | F | F | H | F | F | H | K 157 SmA 177 N 203,4 I |
| 110 | H | F | F | H | F | F | K 169 SmA (158) N 196,9 I |
| 111 | H | F | F | F | F | H | K 173 N 212,9 I |

### Beispiele 112 bis 136

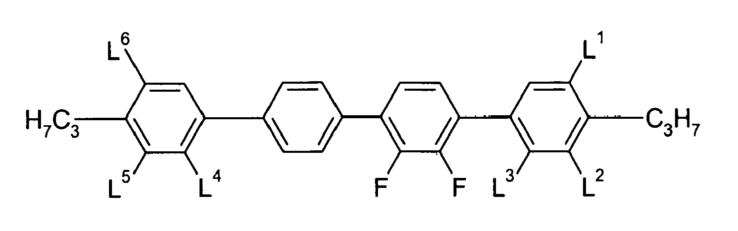

**Tabelle 6**

| Beispiel | L¹ | L² | L³ | L⁴ | L⁵ | L⁶ | Phasenübergänge [°C] |
|---|---|---|---|---|---|---|---|
| 112 | H | H | H | H | H | H | K 131 N 318,0 I |
| 113 | H | F | H | H | H | H | K 97 SmA 227 N 286,1 I |
| 114 | H | H | F | H | H | H | K 131 N 281,2 I |
| 115 | H | H | H | F | H | H | K 136 N 274,2 I |
| 116 | H | H | H | H | F | H | K 109 SmA 216 N 280,9 I |
| 117 | F | F | H | H | H | H | K 94 SmA 251 N 261,8 I |
| 118 | H | F | F | H | H | H | K 130 SmA (113) N 271,8 I |
| 119 | H | H | H | F | F | H | K 139 N 276,8 I |
| 120 | H | H | H | H | F | F | K 146 SmA 234 N 252,5 I |
| 121 | H | F | H | H | F | H | K 114 SmA 233 N 262,8 I |
| 122 | H | F | H | F | H | H | K 131 N 233,4 I |
| 123 | H | H | F | H | F | H | K 130 N 245,2 I |
| 124 | H | H | F | F | H | H | K 160 N 241,8 I |
| 125 | F | F | H | F | H | H | K 148 SmA 177 N 214,6 I |
| 126 | F | F | H | H | F | H | K 113 SmA 225 N 235,4 I |
| 127 | H | F | F | F | H | H | K 149 N 235,7 I |
| 128 | H | F | F | H | F | H | K 144 N 242,3 I |
| 129 | H | F | H | H | F | F | K 139 SmA 228 N 234,8 I |
| 130 | H | H | F | H | F | F | K 152 SmA 176 N 212,6 I |
| 131 | H | F | H | F | F | H | K 151 SmA 176 N 250,7 I |
| 132 | H | H | F | F | F | H | K 162 N 240,1 I |
| 133 | F | F | H | H | F | F | K 152 SmA 208 N 212,6 I |
| 134 | F | F | H | F | F | H | K 151 SmA 204 N 223,2 I |
| 135 | H | F | F | H | F | F | K 156 SmA 179 N 210,7 I |
| 136 | H | F | F | F | F | H | K 172 N 235,6 I |

### Beispiele 137 bis 151

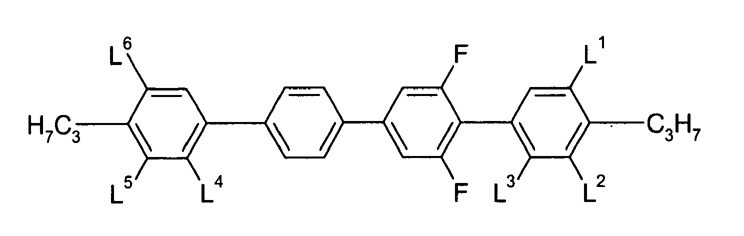

**Tabelle 7**

| Beispiel | L¹ | L² | L³ | L⁴ | L⁵ | L⁶ | Phasenübergänge [°C] |
|---|---|---|---|---|---|---|---|
| 137 | H | H | H | H | H | H | |
| 138 | H | F | H | H | H | H | K 148 SmA 236 N 274,5 I |
| 139 | H | H | H | F | H | H | K 132 N 257,8 I |
| 140 | H | H | H | H | F | H | K 146 SmA 214 N 267,3 I |
| 141 | F | F | H | H | H | H | |
| 142 | H | H | H | F | F | H | K 138 N 251,2 I |
| 143 | H | H | H | H | F | F | K 186 SmA 217 N 236,3 I |
| 144 | H | F | H | H | F | H | |
| 145 | H | F | H | F | H | H | K 133 SmA 154 N 238,9 I |
| 146 | F | F | H | F | H | H | |
| 147 | F | F | H | H | F | H | |
| 148 | H | F | H | H | F | F | K 154 SmA 198 N 219,5 I |
| 149 | H | F | H | F | F | H | K 141 SmA 173 N 229,5 I |
| 150 | F | F | H | H | F | F | |
| 151 | F | F | H | F | F | H | |

### Beispiele 152 bis 176

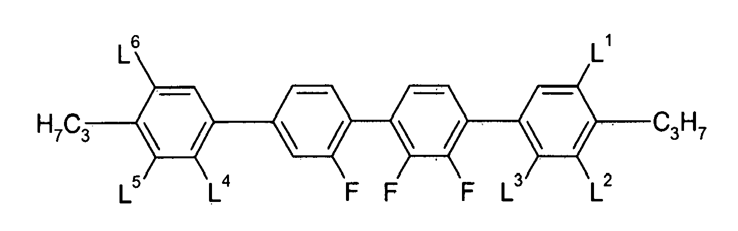

**Tabelle 8**

| Beispiel | L¹ | L² | L³ | L⁴ | L⁵ | L⁶ | Phasenübergänge [°C] |
|---|---|---|---|---|---|---|---|
| 152 | H | H | H | H | H | H | K 150 Sm? (137) N 267,7 I |
| 153 | H | F | H | H | H | H | K 153 N 240,5 I |
| 154 | H | H | F | H | H | H | K 172 N 240,5 I |
| 155 | H | H | H | F | H | H | K 176 N 243,2 I |
| 156 | H | H | H | H | F | H | K 157 N 214,9 I |
| 157 | F | F | H | H | H | H | K 161 SmA 166 N 207,1 I |
| 158 | H | F | F | H | H | H | K 178 N 232,5 I |
| 159 | H | H | H | F | F | H | K 173 N 203,1 I |
| 160 | H | H | H | H | F | F | K 160 N 203,5 I |
| 161 | H | F | H | H | F | H | K 151 N 238,6 I |
| 162 | H | F | H | F | H | H | K 174 N 211,1 I |
| 163 | H | H | F | H | F | H | K 172 N 210,1 I |
| 164 | H | H | F | F | H | H | K 196 N 211,0 I |
| 165 | F | F | H | F | H | H | K 185 N (181,3) I |
| 166 | F | F | H | H | F | H | K 166 SmA 176 N 193,3 I |
| 167 | H | F | F | F | H | H | K 193 N 206,0 I |
| 168 | H | F | F | H | F | H | K 176 N 198,8 I |
| 169 | H | F | H | H | F | F | K 163 SmA 174 N 193,3 I |
| 170 | H | H | F | H | F | F | K 182 N (178,5) I |
| 171 | H | F | H | F | F | H | K 183 N 208,1 I |
| 172 | H | H | F | F | F | H | K 193 N 206,1 I |
| 173 | F | F | H | H | F | F | K 183 SmA (181) N (182,5) I |
| 174 | F | F | H | F | F | H | K 188 N (183,0) I |
| 175 | H | F | F | H | F | F | K 183 N (173,1) I |
| 176 | H | F | F | F | F | H | |

### Beispiele 177 bis 201

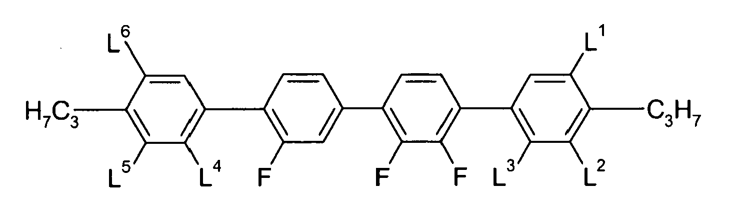

**Tabelle 9**

| Beispiel | L¹ | L² | L³ | L⁴ | L⁵ | L⁶ | Phasenübergänge [°C] |
|---|---|---|---|---|---|---|---|
| 177 | H | H | H | H | H | H | K 144 N 281,2 I |
| 178 | H | F | H | H | H | H | K 143 SmA 160 N 256,2 I |
| 179 | H | H | F | H | H | H | K 164 N 247,3 I |
| 180 | H | H | H | F | H | H | K 165 N 249,3 I |
| 181 | H | H | H | H | F | H | K 142 SmA 174 N 256,2 I |
| 182 | F | F | H | H | H | H | K 139 SmA 200 N 226,7 I |
| 183 | H | F | F | H | H | H | K 162 N 241,7 I |
| 184 | H | H | H | F | F | H | K 169 N 241,2 I |
| 185 | H | H | H | H | F | F | K 144 SmA 207 N 226,8 I |
| 186 | H | F | H | H | F | H | K 150 SmA 194 N 234,7 I |
| 187 | H | F | H | F | H | H | K 161 N 222,2 I |
| 188 | H | H | F | H | F | H | K 162 N 222,2 I |
| 189 | H | H | F | F | H | H | K 182 N 216,3 I |
| 190 | F | F | H | F | H | H | K 173 N 192,8 I |
| 191 | F | F | H | H | F | H | K 154 SmA 193 N 209,0 I |
| 192 | H | F | F | F | H | H | K 178 N 209,1 I |
| 193 | H | F | F | H | F | H | K 169 N 216,8 I |
| 194 | H | F | H | H | F | F | K 157 SmA 197 N 212,6 I |
| 195 | H | H | F | H | F | F | K 169 N 193,7 I |
| 196 | H | F | H | F | F | H | K 172 N 217,3 I |
| 197 | H | H | F | F | F | H | K 179 N 206,1 I |
| 198 | F | F | H | H | F | F | K 179 SmA 181 N 192,8 I |
| 199 | F | F | H | F | F | H | K 175 N 190,5 I |
| 200 | H | F | F | H | F | F | K 176 N 190,4 I |
| 201 | H | F | F | F | F | H | K 188 N 201,9 I |

### Beispiele 202 bis 216

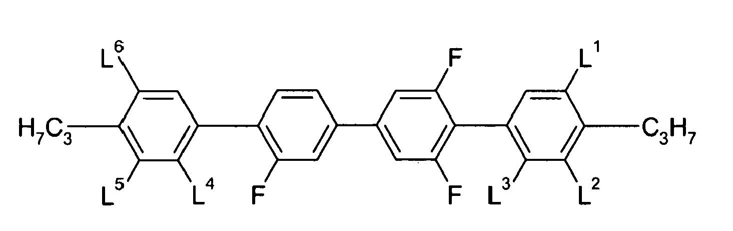

**Tabelle 10**

| Beispiel | L¹ | L² | L³ | L⁴ | L⁵ | L⁶ | Phasenübergänge [°C] |
|---|---|---|---|---|---|---|---|
| 202 | H | H | H | H | H | H | |
| 203 | H | F | H | H | H | H | |
| 204 | H | H | H | F | H | H | |
| 205 | H | H | H | H | F | H | |
| 206 | F | F | H | H | H | H | |
| 207 | H | H | H | F | F | H | |
| 208 | H | H | H | H | F | F | |
| 209 | H | F | H | H | F | H | |
| 210 | H | F | H | F | H | H | |
| 211 | F | F | H | F | H | H | |
| 212 | F | F | H | H | F | H | |
| 213 | H | F | H | H | F | F | |
| 214 | H | F | H | F | F | H | |
| 215 | F | F | H | H | F | F | |
| 216 | F | F | H | F | F | H | |

### Beispiele 217 bis 231

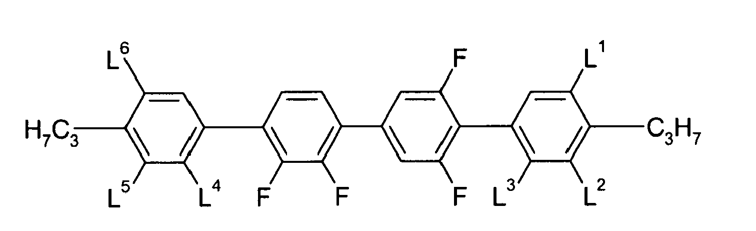

**Tabelle 11**

| Beispiel | L¹ | L² | L³ | L⁴ | L⁵ | L⁶ | Phasenübergänge [°C] |
|---|---|---|---|---|---|---|---|
| 217 | H | H | H | H | H | H | K 172 N 249,0 I |
| 218 | H | F | H | H | H | H | K 168 N 228,9 I |
| 219 | H | H | H | F | H | H | K 185 N 216,2 I |
| 220 | H | H | H | H | F | H | K 169 N 224,0 I |
| 221 | F | F | H | H | H | H | - |
| 222 | H | H | H | F | F | H | K 185 N 210,6 I |
| 223 | H | H | H | H | F | F | K 174 SmA (165) N 199,1 I |
| 224 | H | F | H | H | F | H | K 172 SmA (166) N 210,8 I |
| 225 | H | F | H | F | H | H | K 182 N 195,5 I |
| 226 | F | F | H | F | H | H | K 186 I |
| 227 | F | F | H | H | F | H | K 182 SmA 194 N 240,6 I |
| 228 | H | F | H | H | F | F | K 181 SmA ? N 190,3 I |
| 229 | H | F | H | F | F | H | K 186 N 192,0 I |
| 230 | F | F | H | H | F | F | K 194 I |
| 231 | F | F | H | F | F | H | K 192 I |

### Beispiele 232 bis 247

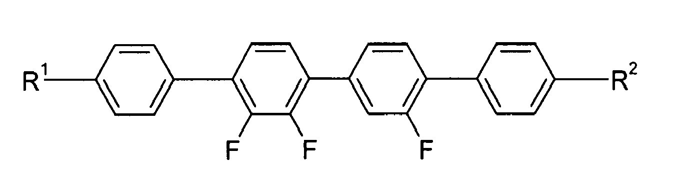

**Tabelle 12**

| Beispiel | R¹ | R² | Phasenübergänge [°C] |
|---|---|---|---|
| 232 | C₃H₇ | C₃H₇ | |
| 233 | C₃H₇ | C₄H₉ | |
| 234 | C₃H₇ | C₅H₁₁ | |
| 235 | C₃H₇ | C₆H₁₃ | |
| 236 | C₄H₉ | C₃H₇ | K 127 N 263,6 I |
| 237 | C₄H₉ | C₄H₉ | K 124 N 251,7 I |
| 238 | C₄H₉ | C₅H₁₁ | |
| 239 | C₄H₉ | C₆H₁₃ | |
| 240 | C₅H₁₁ | C₃H₇ | |
| 241 | C₅H₁₁ | C₄H₉ | |
| 242 | C₅H₁₁ | C₅H₁₁ | |
| 243 | C₅H₁₁ | C₆H₁₃ | |
| 244 | C₆H₁₃ | C₃H₇ | |
| 245 | C₆H₁₃ | C₄H₉ | |
| 246 | C₆H₁₃ | C₅H₁₁ | |
| 247 | C₆H₁₃ | C₆H₁₃ | |

### Beispiele 248 bis 272

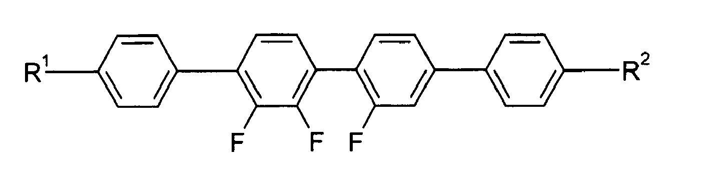

**Tabelle 13**

| Beispiel | R¹ | R² | Phasenübergänge [°C] |
|---|---|---|---|
| 248 | C₃H₇ | C₃H₇ | |
| 249 | C₃H₇ | t-C₄H₉ | |
| 250 | C₃H₇ | C₅H₁₁ | K 99 N 250,6 I |
| 251 | C₃H₇ | C₆H₁₃ | |
| 252 | C₃H₇ | C₁₀H₂₁ | K 93 N 207,9 I |
| 253 | t-C₄H₉ | C₃H₇ | |
| 254 | t-C₄H₉ | t-C₄H₉ | |
| 255 | t-C₄H₉ | C₅H₁₁ | K 133 N 140,8 I |
| 256 | t-C₄H₉ | C₆H₁₃ | |
| 257 | t-C₄H₉ | C₁₀H₂₁ | K 115 N 119,3 I |
| 258 | C₅H₁₁ | C₃H₇ | K 103 N 252,5 I |
| 259 | C₅H₁₁ | t-C₄H₉ | K 134 N 144,3 I |
| 260 | C₅H₁₁ | C₅H₁₁ | K 106 N 234,4 I |
| 261 | C₅H₁₁ | C₆H₁₃ | |
| 262 | C₅H₁₁ | C₁₀H₂₁ | K 85 Sm?; N 198,7 I |
| 263 | C₆H₁₃ | C₃H₇ | |
| 264 | C₆H₁₃ | t-C₄H₉ | |
| 265 | C₆H₁₃ | C₅H₁₁ | |
| 266 | C₆H₁₃ | C₆H₁₃ | |
| 267 | C₆H₁₃ | C₁₀H₂₁ | |
| 268 | C₁₀H₂₁ | C₃H₇ | K 103 SmC 114 N 205,1 I |
| 269 | C₁₀H₂₁ | t-C₄H₉ | K 101 SmC 123 N 127,5 I |
| 270 | C₁₀H₂₁ | C₅H₁₁ | K 102 SmC 135 N 197,5 I |
| 271 | C₁₀H₂₁ | C₆H₁₃ | |
| 272 | C₁₀H₂₁ | C₁₀H₂₁ | K 104 SmC 158 N 177,3 I |

### Beispiel 273

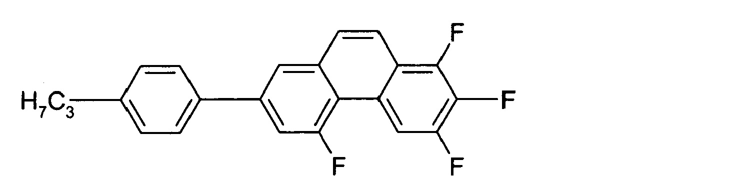

Phasenübergänge [°C]: K 108 SmA 109 N 133,1 I

### Beispiel 274

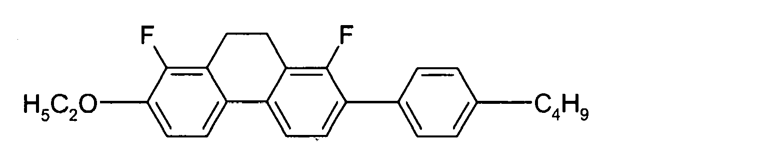

Phasenübergänge [°C]: K 113 SmA 141 N 204,0 I

### Beispiel 275

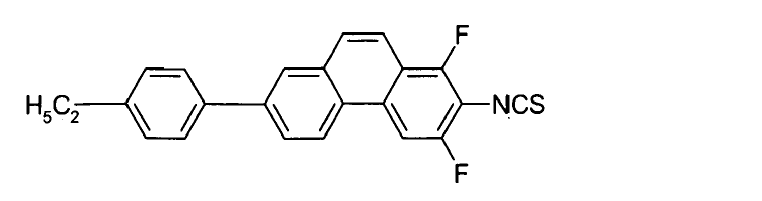

Phasenübergänge [°C]: K 127 N

### Beispiel 276

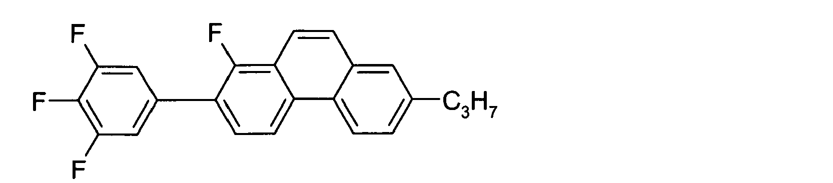

Phasenübergänge [°C]: K 114 SmC 136 I

### Beispiel 277

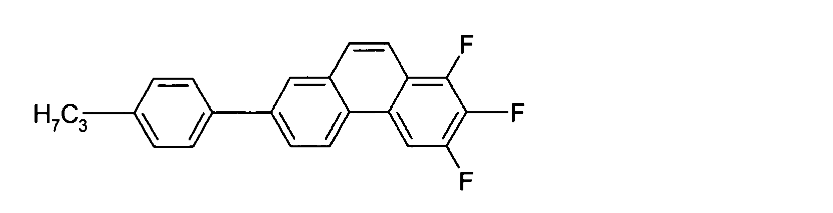

Phasenübergänge [°C]: K 131 SmA 143 I

### Beispiel 278

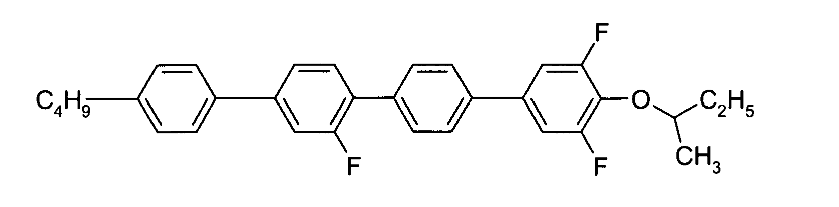

Phasenübergänge [°C]: K 138 SmA 244 I

Zu einer flüssigkristallinen Verbindung der folgenden Formel: 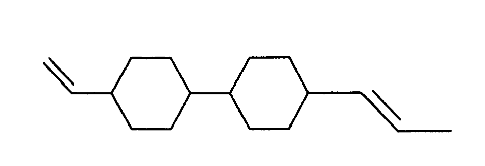 wird 1 Gew.-% der Verbindung des Beispiels 278 hinzugegeben und die Drehkraft HTP der Zusammensetzung nach der Grandjean-Cano Methode bei 20°C bestimmt. Die Zusammensetzung weist eine HTP von 3,4 auf.

### Beispiel 279

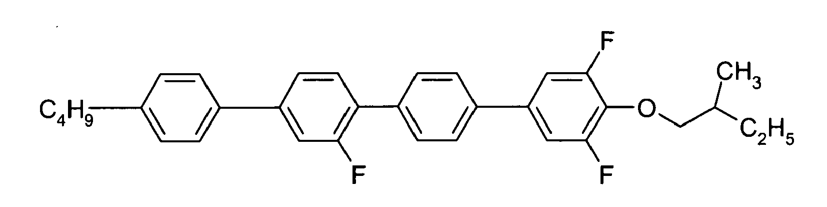

Phasenübergänge [°C]: K 111 SmA 264 I

Zu einer flüssigkristallinen Mischung, die die folgenden Verbindungen enthält: 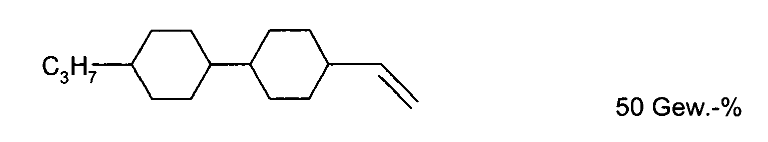 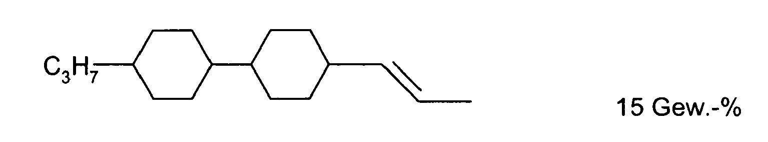 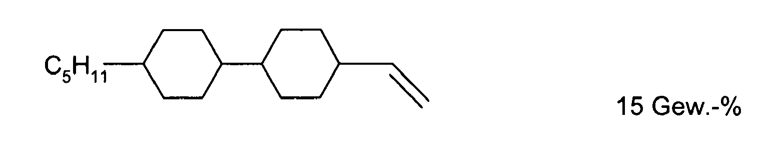 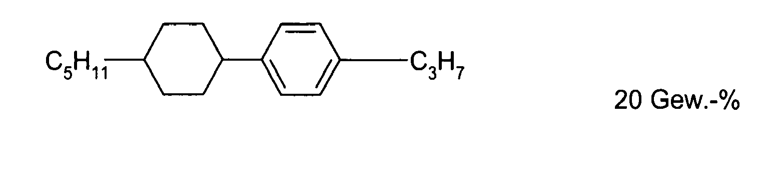 wird 1 Gew.-% der Verbindung des Beispiels 279 hinzugegeben und die Drehkraft HTP der Zusammensetzung nach der Grandjean-Cano Methode bei 20°C bestimmt. Die Zusammensetzung weist eine HTP von 6,3 auf.

### Beispiel 280

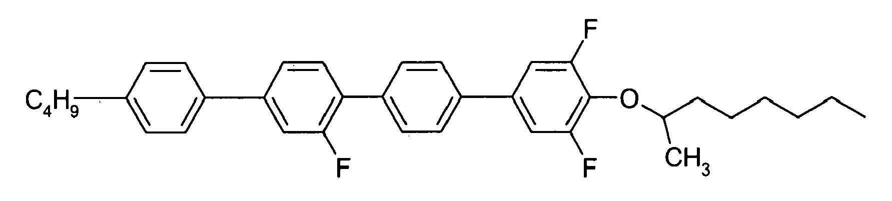

Phasenübergänge [°C]: K 108 SmA 216 I

Zu einer flüssigkristallinen Verbindung der folgenden Formel: 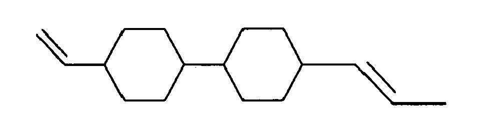 wird 1 Gew.-% der Verbindung des Beispiels 280 hinzugegeben und die Drehkraft HTP der Zusammensetzung nach der Grandjean-Cano Methode bei 20°C bestimmt. Die Zusammensetzung weist eine HTP von 6,7 auf.

### Beispiel 281

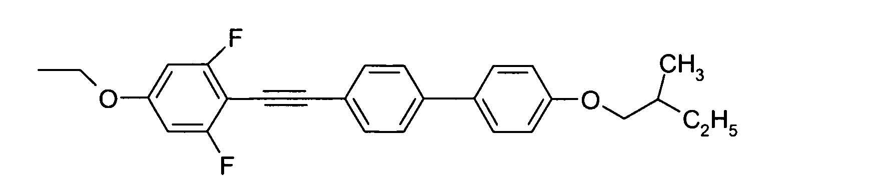

Phasenübergänge [°C]: K 97 N 190,3 I

Zu einer flüssigkristallinen Verbindung der folgenden Formel: 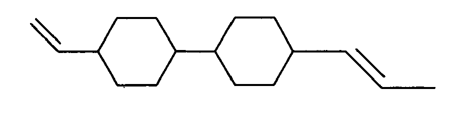 wird 1 Gew.-% der Verbindung des Beispiels 281 hinzugegeben und die Drehkraft HTP der Zusammensetzung nach der Grandjean-Cano Methode bei 20°C bestimmt. Die Zusammensetzung weist eine HTP von 0,8 auf.

### Beispiel 282

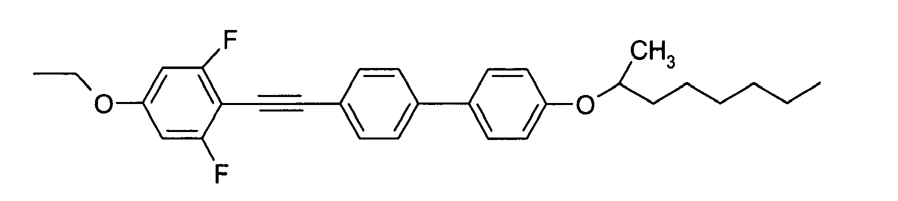

Phasenübergänge [°C]: K 92 N 112 I

Zu einer flüssigkristallinen Mischung, wie in Beispiel 279 offenbart, wird 1 Gew.-% der Verbindung des Beispiels 282 hinzugegeben und die Drehkraft HTP der Zusammensetzung nach der Grandjean-Cano Methode bei 20°C bestimmt. Die Zusammensetzung weist eine HTP von 1,3 auf.

## Patentansprüche

1. Ringverbindungen der allgemeinen Formel XIII 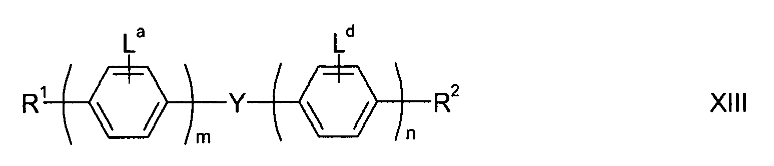 wobei
m und n unabhängig voneinander, gleich oder verschieden 0 oder 1 bedeuten und die Summe (m + n) = 1 oder 2 ist,
Y eine Gruppe der Formel XIV bis XVII darstellt 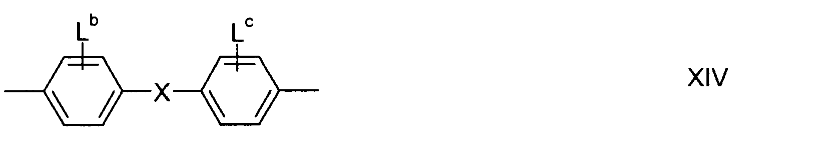 mit X = Einfachbindung, -CH₂-CH₂-, -CH=CH-, -C≡C- oder 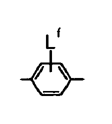 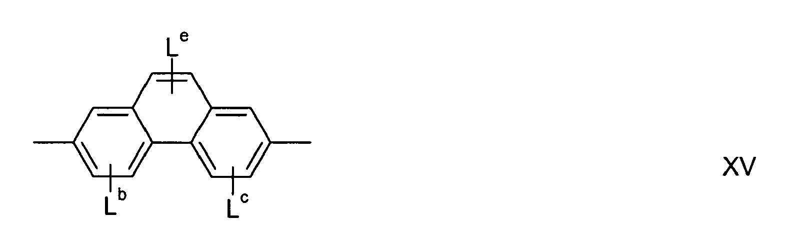 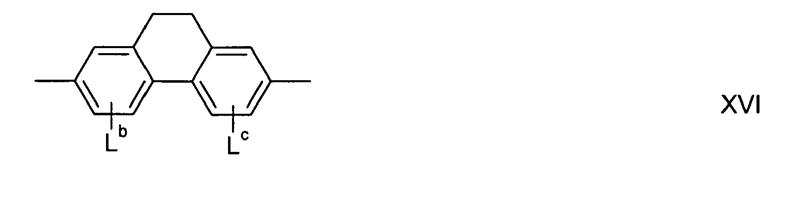 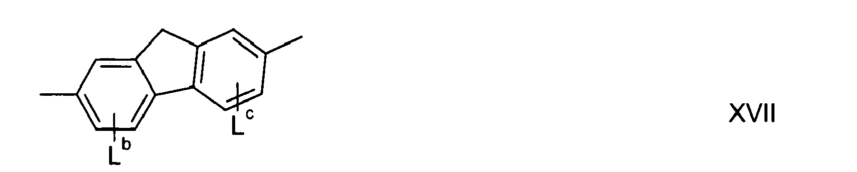
L unabhängig voneinander, gleich oder verschieden R, F, Cl, Br, I, OH, OR, SH, SR, CN, NO₂, NO, CHO, COOH, COOR, CONH₂, CONHR, CONR₂, CF₃, NH₂, NHR oder NR₂, wobei R eine Alkyl-, eine Alkenyl- bzw. eine Acylgruppe mit 1 bis 12 C-Atomen oder eine Arylgruppe mit 6 C-Atomen, die gegebenenfalls wiederum durch eine Alkylgruppe mit 1 bis 12 C-Atomen substituiert sein kann, und
a, b, c, d, e und f unabhängig voneinander, gleich oder verschieden 0, 1, 2 und die Summe (a+b+c+d+e+f) = 1 bis 8, vorzugsweise 3 bis 8,
bedeuten,
in den aromatischen Ringsystemen eine oder zwei CH-Gruppen durch N ersetzt sein können,
R¹ und R² unabhängig voneinander, gleich oder verschieden H, F, CI, CN, NCS oder einen geradkettigen oder verzweigten, gegebenenfalls chiralen Alkylrest oder Alkoxyrest mit 1 bis 12 C-Atomen bzw. einen Alkenylrest oder Alkinylrest mit 2 bis 8 C-Atomen bedeuten, worin auch jeweils eine CH₂-Gruppe durch -O-, -CO-, -O-CO-, -COO- oder -CH=CH- so ersetzt sein kann, dass Heteroatome nicht direkt miteinander verknüpft sind und/oder ein oder mehrere H durch Halogen, vorzugsweise F, ersetzt sein können, wobei mindestens einer der beiden Reste einen
verzweigten, chiralen Alkylrest oder Alkoxyrest mit 1 bis 12 C-Atomen bzw. einen chiralen Alkenylrest oder Alkinylrest mit 2 bis 8 C-Atomen bedeutet, worin auch jeweils eine CH₂-Gruppe durch -O-, -CO-, -O-CO-, -COO- oder -CH=CH- so ersetzt sein kann, dass Heteroatome nicht direkt miteinander verknüpft sind und/oder ein oder mehrere H durch Halogen, vorzugsweise F, ersetzt sein können, und
wobei für den Fall, dass m = 0 oder n = 0 in R¹ oder R² eine CH₂-Gruppe durch eine der folgenden Gruppen ersetzt sein kann:
a) trans-1,4-Cyclohexylen, worin auch eine oder mehrere nicht benachbarte CH₂-Gruppen durch -O- und/oder -S- ersetzt sein können,
b) einen Rest aus der Gruppe 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl oder
c) 1,4-Cyclohexenylen,
und worin die Reste a), b), c) auch durch CN und/oder Halogen substituiert sein können.

2. Ringverbindungen gemäß Anspruch 1, **gekennzeichnet durch** die allgemeine Formel XVIII 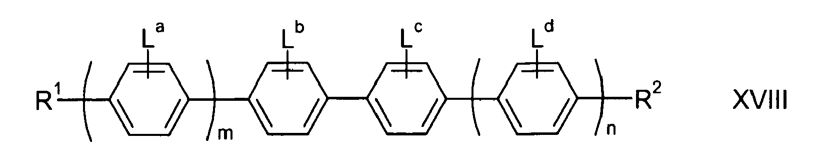 wobei m=n=1.

3. Ringverbindungen gemäß Anspruch 1 oder 2, **dadurch gekennzeichnet, dass** R¹ und R² unabhängig voneinander, gleich oder verschieden, ausgewählt sind aus der Gruppe C₁-C₇-Alkyl.
